# EUROPEAN PATENT APPLICATION

(11) **EP 4 813 989 A1**
(43) Date of publication of application: **30.09.2026**
(21) Application number: 23959268.6
(22) Date of filing: 24.11.2023
(51) Int. Cl.: C07D 487/04, A61K 31/519, A61K 31/41, A61P 35/00

(54) **CRYSTAL, PHARMACEUTICAL COMPOSITION, PREPARATION METHOD THEREFOR, AND USE THEREOF**

(71) Applicant: TYK Medicines, Inc., Huzhou, Zhejiang 313100 (CN); TYK Medicines (Zhengzhou), Inc., Zhengzhou, Henan 451162 (CN)
(72) Inventor: NIU, Chengshan, Zhengzhou, Henan 451162 (CN); ZHENG, Maolin, Zhengzhou, Henan 451162 (CN); WANG, Guohui, Zhengzhou, Henan 451162 (CN); LIANG, Apeng, Huzhou, Zhejiang 313100 (CN); CHEN, Shaoqing, Huzhou, Zhejiang 313100 (CN); LI, Jun, Huzhou, Zhejiang 313100 (CN); WU, Yusheng, Huzhou, Zhejiang 313100 (CN)
(74) Representative: Engelhard, Markus
(86) International application number: PCT/CN2023/134036
(87) International publication number: WO 2025/107298

(57) **Abstract**

The present disclosure relates to a pharmaceutical composition of a crystal form A of a compound represented by formula I. The composition has excellent storage stability and dissolution performance, and the crystal form A has good stability, reduced hygroscopicity and good bioavailability. The pharmaceutical composition and the crystal form A have very important significance for improving the production and quality control of drugs, the development prospect of solid oral formulation, and the like.

## Description

### TECHNICAL FIELD

The present disclosure belongs to the technical field of medicinal chemistry, and particularly relates to a crystal form of a compound used as a kinase inhibitor, and a preparation method therefor and use thereof.

### BACKGROUND

A tropomyosin receptor kinase (TRK) family belongs to transmembrane receptor tyrosine kinases (RTKs), and is involved in regulation of synapse growth and functional maintenance of mammalian nervous system, occurrence and development of memory, protection of neurons from injury, and the like. TRK kinases are a type of nerve growth factor receptors, and the family consists of a highly homologous tropomyosin-related kinase A (TRKA), a tropomyosin-related kinase B (TRKB) and a tropomyosin-related kinase C (TRKC), which are encoded by NTRK1, NTRK2 and NTRK3 genes respectively. An intact TRK kinase comprises three parts: an extracellular domain, a transmembrane domain and an intracellular domain. Similar to other RTKs, after binding the extracellular domain of the TRK kinase to a corresponding ligand, a dimer is formed, which can induce autophosphorylation of the intracellular domain of the TRK kinase, thereby activating the kinase activity and further activating a downstream signal transduction pathway. The TRK kinase affects cell proliferation, differentiation, metabolism and apoptosis through downstream pathways such as Ras/MAPK, PI3K/AKT and PLCγ. In the case of NTRKs gene fusion or mutation, a receptor of the extracellular domain may be altered or eliminated (Greco, A. et al, Mol. Cell. Biol. 1995, 15, 6118; Oncogene 1998, 16, 809), and a fused or mutated TRK protein is in a highly activated kinase state without ligand binding, thereby continuously activating the downstream signal transduction pathway, leading to dysregulation of the downstream signaling pathway of the TRK kinase, inducing cell proliferation, and promoting tumor occurrence and development. The NTRKs gene fusion occurs in a variety of adult and pediatric solid tumors, including breast cancer, colorectal cancer, non-small cell lung cancer, papillary thyroid cancer, Spitzoid melanoma, glioma, and various sarcomas. In common cancers, such as non-small cell lung cancer and colorectal cancer, an incidence rate of the NTRKs gene fusion is relatively low, approximately 1%-3%. However, in some rare cancers, such as infantile fibrosarcoma and mammary secretory carcinoma, the incidence rate of the NTRKs gene fusion can reach more than 90%. The first TPM3-TRKA fusion protein was discovered in colon cancer cells. Subsequently, more types of NTRK fusion proteins, such as CD74-NTRKA, MPRIP-NTRKA, QKI-NTRKB, ETV6-NTRKC and BTB1-NTRKC, were found in different clinical tumor patient samples including breast cancer, non-small cell lung cancer, papillary thyroid cancer, Spitzoid melanoma, glioma and the like. Therefore, in recent years, the NTRK fusion proteins have become an effective anti-cancer target and a hotspot in the research and development of anti-cancer drugs. With people's further in-depth understanding of the TRK kinases in recent years, more types of TRK fusion proteins and mutations have been found (Russo, M. et. al Cancer; Discovery, 2016, 6, 36; Drilon, A. et al, Annals of Oncology, 2016, 27, 920). Therefore, there is an urgent clinical need to develop novel NTRK inhibitors with better activity and wider application, so as to solve the problem with the treatment of tumors caused by the NTRK protein fusion or mutation.

ROS1 (c-ros oncogene 1 receptor kinase) is a tyrosine protein kinase encoded by an ROS1 proto-oncogene in the human body, which is positioned on a chromosome 6q22.1, belongs to a tyrosine kinase insulin receptor gene, consists of 3 parts: an intracellular tyrosine kinase active domain, a transmembrane domain and an extracellular domain, and encodes a chimeric protein with tyrosine kinase activity. The basic structure is composed of an extracellular N-terminal ligand-binding domain (amino acids 1-1861), a transmembrane domain (amino acids 1862-1882) and an intracellular C-terminal tyrosine kinase active domain consisting of 464 amino acids (amino acids 1883-2347). During ROS1 gene rearrangement, the extracellular domain is lost, while the transmembrane domain and the intracellular tyrosine kinase domain are retained, and rearrangement sites mainly occur in exons 32-36 of the ROS1 gene. ROS1 gene mutation mainly occurs in lung cancer patients, and a proportion of the patients is 1%-2%. In NSCLC, the ROS1 gene is mainly fused with SLC34A2 and CD74, and continuously activates the ROS1 tyrosine kinase domain and the downstream signal pathways including JAK/STAT, PI3K/AKT, RAS/MAPK, and the like, thereby inducing tumorigenesis. It has been confirmed in numerous literatures and clinical practice that, diseases caused by overactivation of ROS1, especially cancer, can be treated by inhibiting the activity of mutated ROS1 kinase. Currently marketed therapeutic drugs for ROS1-positive non-small cell lung cancer include crizotinib and entrectinib, both of which are first-generation small-molecule ROS1 inhibitors. However, during treatment with crizotinib or entrectinib, drug resistance usually develops after approximately 15 months, leading to disease progression. Among patients with drug resistance, the most common resistance mutation is solvent front mutation, such as G2032R, and no therapeutic drug is currently marketed for the patients with drug resistance. Therefore, there is an urgent need to develop novel inhibitors targeting the ROS1, especially novel ROS1 inhibitor drugs for clinical treatment of patients with resistance to first-generation ROS1 inhibitors such as crizotinib and entrectinib.

About 2-5% of NSCLC cases belong to an anaplastic lymphoma kinase (ALK) rearrangement type, and the anaplastic lymphoma kinase is a receptor-type protein tyrosine kinase of an insulin receptor superfamily. The ALK was initially discovered as an activated fusion oncogene in anaplastic large cell lymphoma, and ALK fusion forms have been found in a variety of cancers through subsequent successive studies, comprising systemic histiocytic hyperplasia, inflammatory myofibroblastic tumor, non-small cell lung cancer, and the like. Due to mutations and abnormal activity of the ALK in a variety of cancers, the ALK has become a drug target for treating ALK-positive cancers. A number of ALK kinase inhibitors are currently marketed. With the clinical application of these drugs, patients will develop drug resistance mutations, such as G1202R, resulting in loss of efficacy of these drugs.

With people's further in-depth understanding of kinases such as ROS1, NTRK and ALK in recent years, and the increasing number of clinical patients with drug resistance, there is an urgent clinical need to develop novel tyrosine kinase inhibitors with better activity and wider applications, so as to solve the problem with the treatment of tumors caused by fusion or mutation of kinase proteins such as ROS1, NTRK and ALK.

The patent CN112867717A discloses a kinase inhibitor capable of simultaneously acting on oncoproteins including NTRK, ALK and/or ROS1 - a compound TY-2136b represented by formula I below, the chemical name of the free base of the compound is: (R)-3-(5,5-dimethyl-4,5-dihydro-1,2,4-oxadiazol-3-yl)-N-(1-(2,3,5-trifluorophenyl)ethyl)pyrazolo[1 ,5-a]pyrimidin-5-amine:

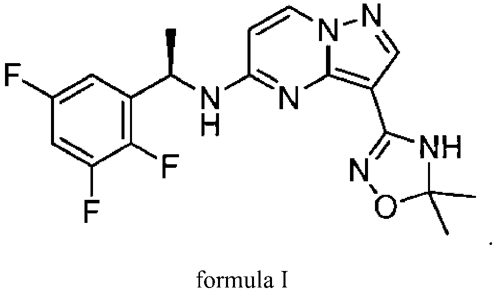

Drugs need to be in a solution to be absorbed, and such precipitation may affect an absorption extent and an absorption rate of the drug. A compound with pH-dependent solubility (particularly a basic compound) may show undesirable pharmacokinetic properties, such as poor absorption or low bioavailability, which may lead to significant inter-patient and intra-patient variability. Therefore, there is a need to find an improved dosage form of TY-2136b with a favorable dissolution and pharmacokinetics curve and good storage stability.

There has been no report on the preparation of a formulation of the compound represented by formula I above. In order to deliver therapeutic benefits of the TY-2136b to patients in need thereof, it is necessary to prepare the TY-2136b into a pharmaceutical composition, particularly a solid dosage form suitable for oral administration. Therefore, there is a need for a stable pharmaceutical formulation of the TY-2136b with good in-vivo dissolution, high bioavailability, and good storage stability and accelerated stability.

In addition, no literature has been reported on the preparation of a crystal form of the compound represented by formula I above. It is known to those skilled in the art that the discovery of a compound form favorable for purification and quality control of a pharmaceutical compound is of great significance for improving properties such as drug production, quality control and a development prospect of solid oral formulation. Because different crystal forms of a drug may affect formulation processability, bioavailability and therapeutic efficacy of the drug, the research on the crystal form of the drug is of great importance. Therefore, the properties of the TY-2136b, such as formulation processability including stability and flowability, quality control and bioavailability, still need to be improved, so that there is a need to develop a suitable form of the compound above and a preparation method therefor.

### SUMMARY

One or more embodiments of the present disclosure provide a pharmaceutical composition, which comprises:
a crystal form A of a monohydrate of a compound represented by formula I 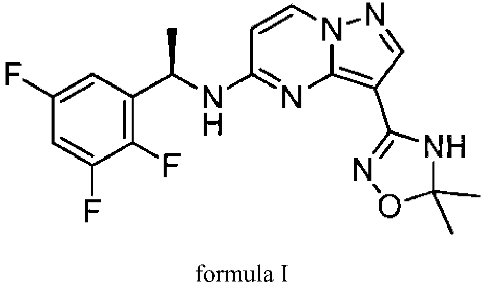
and a pharmaceutically acceptable carrier, adjuvant or excipient;
wherein, the crystal form A has one or more characteristic peaks at 9.35±0.2°, 11.42±0.2°, 12.06±0.2°, 18.71±0.2° and 21.16±0.2° in an X-ray powder diffraction pattern expressed in terms of 2θ angles.

In one or more embodiments, the crystal form A has one or more characteristic peaks at 9.97±0.2°, 13.16±0.2°, 19.15±0.2°, 19.97±0.2° and 21.00±0.2° in the X-ray powder diffraction pattern expressed in terms of 2θ angles.

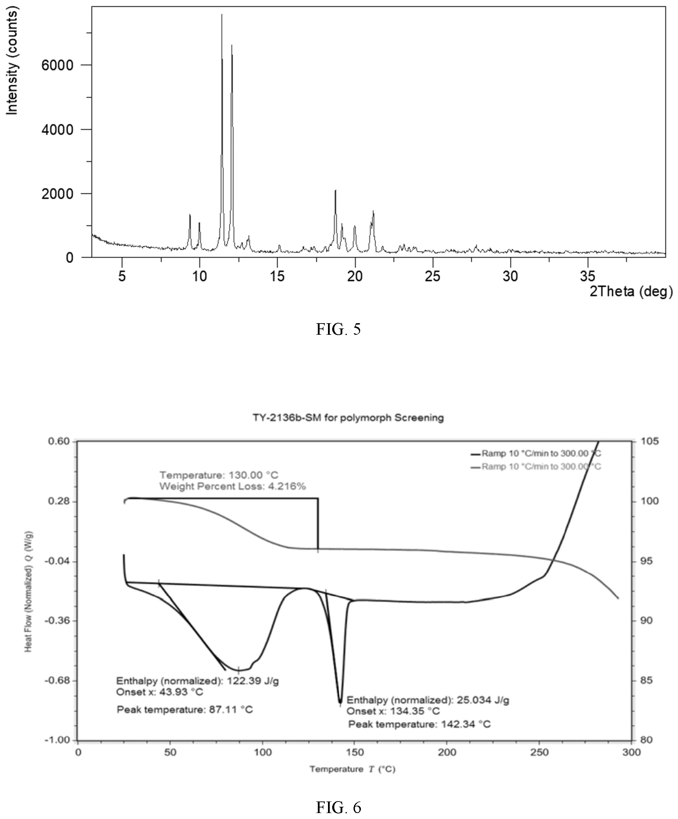

In one or more embodiments, the crystal form A has the X-ray powder diffraction pattern substantially as shown in FIG. 5.

In one or more embodiments, the pharmaceutical composition is prepared as an oral formulation.

In one or more embodiments, the crystal form A has a thermogravimetric analysis thermogram and a differential scanning calorimetry thermogram substantially as shown in FIG. 6.

In one or more embodiments, the crystal form A belongs to a monoclinic system and belongs to a space group P2(1), with unit cell parameters: a=10.2499(10) Å, b=17.9931(16) Å, c= 11.2407(8) Å, α=γ=90° β=93.349(8)°, deviation factor R1=0.0562, and Z=4.

In one or more embodiments,
the pharmaceutical composition comprises the following components in parts by weight:

| Component | Parts by weight |
|---|---|
| Crystal form A | 2.5-40 |
| First diluent | 10-70 |
| Second diluent | 10-70 |
| Glidant | 0.5-5 |
| Disintegrant | 1-15 |
| Lubricant | 0.2-10 |
| Coating material | 1-10 |

In one or more embodiments, D90 of the crystal form A is 17-523 µm, such as 17-191 µm.

In one or more embodiments, the first diluent is selected from mannitol, lactose, anhydrous calcium hydrogen phosphate, or a combination thereof.

In one or more embodiments, the second diluent is microcrystalline cellulose.

In one or more embodiments, the glidant is colloidal silicon dioxide.

In one or more embodiments, the disintegrant is selected from sodium carboxymethyl starch, croscarmellose sodium, crospovidone, or a combination thereof.

In one or more embodiments, the lubricant is selected from sodium stearyl fumarate, magnesium stearate, or a combination thereof.

In one or more embodiments, the coating material comprises a stabilizing substance and a polymer, wherein the stabilizing substance is at least one selected from titanium dioxide, talc and yellow iron oxide, and the polymer is at least one selected from polyvinyl alcohol and polyethylene glycol.

In one or more embodiments, the pharmaceutical composition has one or more of the following characteristics:
1) a dissolution of the crystal form A in the pharmaceutical composition is ≥70% within 15 minutes under conditions of pH 6.0-7.6 and 0.2% SDS;
2) a dissolution of the crystal form A in the pharmaceutical composition is ≥85% within 45 minutes under conditions of pH 6.0-7.6 and 0.2% SDS;
3) a dissolution of the crystal form A in the pharmaceutical composition is ≥95% within 60 minutes under conditions of pH 6.0-7.6 and 0.2% SDS;
4) a dissolution of the crystal form A in the pharmaceutical composition is ≥85% within 45 minutes after the pharmaceutical composition is stored for 18 months under conditions of 25±2°C and 60%±5%RH; and
5) a dissolution of the crystal form A in the pharmaceutical composition is ≥85% within 45 minutes after the pharmaceutical composition is stored for 6 months under conditions of 40±2°C and 75%±5%RH.

One or more embodiments of the present disclosure provide a preparation method for the pharmaceutical composition of the present disclosure, which comprises:
providing the following materials as raw materials in parts by weight, and preparing the raw materials into the composition:

| Component | Parts by weight |
|---|---|
| Crystal form A | 2.5-40 |
| First diluent | 10-70 |
| Second diluent | 10-70 |
| Glidant | 0.5-5 |
| Disintegrant | 1-15 |
| Lubricant | 0.2-10 |
| Coating material | 1-10 |

In one or more embodiments,

| Component | Parts by weight |
|---|---|
| Crystal form A | 4-30 |
| First diluent | 20-60 |
| Second diluent | 20-60 |
| Glidant | 1-4 |
| Disintegrant | 2-12 |
| Lubricant | 0.5-10 |
| Coating material | 1.5-8 |

In one or more embodiments, D90 of the crystal form A is 17-523 µm, such as 17-191 µm.

In one or more embodiments, the first diluent is selected from mannitol, lactose, anhydrous calcium hydrogen phosphate, or a combination thereof.

In one or more embodiments, the second diluent is microcrystalline cellulose.

In one or more embodiments, the glidant is colloidal silicon dioxide.

In one or more embodiments, the disintegrant is selected from sodium carboxymethyl starch, croscarmellose sodium, crospovidone, or a combination thereof.

In one or more embodiments, the lubricant is selected from sodium stearyl fumarate, magnesium stearate, or a combination thereof.

In one or more embodiments, the coating material includes a stabilizing substance and a polymer, wherein the stabilizing substance is at least one selected from titanium dioxide, talc and yellow iron oxide, and the polymer is at least one selected from polyvinyl alcohol and polyethylene glycol.

In one or more embodiments, the preparation method for the crystal form A comprises slurrying the compound represented by formula I using a mixed solvent of acetonitrile and water.

In one or more embodiments, the preparation method for the crystal form A comprises slurrying the compound represented by formula I using the mixed solvent of acetonitrile and water for 1-4 days, and centrifuging to collect a crystalline powder solid.

One or more embodiments of the present disclosure provide the use of the pharmaceutical composition of the present disclosure in the manufacture of a medicament for treating and/or preventing cancer or tumor.

In one or more embodiments, the cancer or tumor is selected from a breast cancer, a cervical cancer, a colon cancer, a lung cancer, a gastric cancer, a rectal cancer, a pancreatic cancer, a brain cancer, a skin cancer, an oral cancer, a prostate cancer, a bone cancer, a renal cancer, an ovarian cancer, a bladder cancer, a liver cancer, a fallopian tube tumor, a peritoneal tumor, a melanoma, a glioma, a glioblastoma, a head and neck cancer, a papillary renal tumor, leukemia, a lymphoma, a myeloma and a thyroid tumor.

One or more embodiments of the present disclosure provide the use of the pharmaceutical composition of the present disclosure in the manufacture of a medicament for treating and/or preventing a disease mediated by ROS1, NTRK or ALK.

In one or more embodiments, the disease mediated by ROS1, NTRK or ALK is selected from cancer, sarcoma and pain.

One or more embodiments of the present disclosure provide a pharmaceutical composition, and the pharmaceutical composition comprises the following components:
component 1), a compound represented by formula I 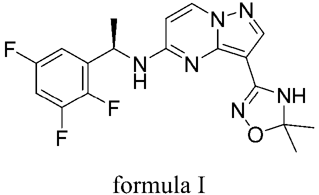
or a pharmaceutically acceptable hydrate, solvate or salt thereof, serving as an active ingredient; and
component 2), a pharmaceutically acceptable carrier.

In one or more embodiments, the pharmaceutical composition comprises the following components in parts by weight:

| Component | Parts by weight | Parts by weight | Parts by weight | Parts by weight |
|---|---|---|---|---|
| Active ingredient | 2.5-40 | 4-30 | 5-20 | 7.5-15 |
| First diluent | 10-70 | 20-60 | 25-55 | 30-50 |
| Second diluent | 10-70 | 20-60 | 25-55 | 30-50 |
| Glidant | 0.5-5 | 1-4 | 1-3 | 1.5-3 |
| Disintegrant | 1-15 | 2-12 | 3-10 | 4-9 |
| Lubricant | 0.2-10 | 0.5-5 | 0.5-3 | 0.5-2 |
| Weight gain of coating | 1-10 | 1.5-8 | 2-7 | 2-6 |

In one or more embodiments, the pharmaceutical composition has one or more characteristics selected from the following cases:
1) a dissolution of the active ingredient in the pharmaceutical composition is ≥70% (such as ≥75%, ≥80% and ≥85%) within 15 minutes under conditions of pH 6.0-7.6 and 0.2% SDS (such as pH 6.2-7.4 or pH 6.5-7.2);
2) a dissolution of the active ingredient in the pharmaceutical composition is ≥85% (such as ≥90% and ≥95%) within 45 minutes under conditions of pH 6.0-7.6 and 0.2% SDS (such as pH 6.2-7.4 or pH 6.5-7.2);
3) a dissolution of the active ingredient in the pharmaceutical composition is ≥95% (such as ≥98% and ≥100%) within 60 minutes under conditions of pH 6.0-7.6 and 0.2% SDS (such as pH 6.2-7.4 or pH 6.5-7.2);
4) a dissolution of the active ingredient in the pharmaceutical composition is ≥85% (such as ≥90% and ≥95%) within 45 minutes after storage for 18 months under conditions of 25±2°C and 60%±5%RH; and
5) a dissolution of the active ingredient in the pharmaceutical composition is ≥85% (such as ≥90% and ≥95%) within 45 minutes after storage for 6 months under conditions of 40±2°C and 75%±5%RH.

In one or more embodiments, the pharmaceutically acceptable compound is a monohydrate.

In one or more embodiments, the pharmaceutical composition is an oral formulation.

In one or more embodiments, the pharmaceutical composition is a solid dosage form.

In one or more embodiments, the pharmaceutical composition is a tablet.

In one or more embodiments, the pharmaceutical composition further comprises a coating material.

In one or more embodiments, a weight of the coating material is 1.5-10%, such as 1.8-8% and 2-6%, of a total weight of the component 1) and the component 2).

One or more embodiments of the present disclosure provide a preparation method for the pharmaceutical composition of the present disclosure, which comprises:
1) providing the following materials as raw materials;

| Component | Parts by weight | Parts by weight | Parts by weight | Parts by weight |
|---|---|---|---|---|
| Active ingredient | 2.5-40 | 4-30 | 5-20 | 7.5-15 |
| First diluent | 10-70 | 20-60 | 25-55 | 30-50 |
| Second diluent | 10-70 | 20-60 | 25-55 | 30-50 |
| Glidant | 0.5-5 | 1-4 | 1-3 | 1.5-3 |
| Disintegrant | 1-15 | 2-12 | 3-10 | 4-9 |
| Lubricant | 0.2-10 | 0.5-5 | 0.5-3 | 0.5-2 |
| Coating material | 1-10 | 1.5-8 | 2-7 | 2-6 |

2) preparing the above materials into the pharmaceutical composition.

In one or more embodiments, the step 2) comprises the following steps:
2-1) premixing the active ingredient, the first diluent, the second diluent, the glidant, the disintegrant and the lubricant to obtain a premix;
2-2) subjecting the premix to dry granulation;
2-3) blending the product obtained in the step 2-2) with the second lubricant to obtain a blend; and
2-4) tableting the blend to obtain an uncoated tablet, which is the pharmaceutical composition.

In one or more embodiments, a hardness degree of the uncoated tablet is 40-150 N, such as 70-130 N and 80-120 N.

In one or more embodiments, the hardness degree of the uncoated tablet is 30-100 N, such as 40-90 N and 50-80 N.

One or more embodiments of the present disclosure provide the use of the pharmaceutical composition of the present disclosure in the manufacture of an anti-tumor medicament.

In one or more embodiments, the tumor is any one of a breast cancer, a cervical cancer, a colon cancer, a lung cancer, a gastric cancer, a rectal cancer, a pancreatic cancer, a brain cancer, a skin cancer, an oral cancer, a prostate cancer, a bone cancer, a renal cancer, an ovarian cancer, a bladder cancer, a liver cancer, a fallopian tube tumor, a peritoneal tumor, a melanoma, a glioma, a glioblastoma, a head and neck cancer, a papillary renal tumor, leukemia, a lymphoma, a myeloma and a thyroid tumor.

One or more embodiments of the present disclosure provide a crystal form A of a monohydrate of a compound represented by formula I, which has one or more characteristic peaks at 9.35±0.2°, 11.42±0.2°, 12.06±0.2°, 18.71±0.2° and 21.16±0.2° in an X-ray powder diffraction pattern expressed in terms of 2θ angles, wherein a chemical structural formula of the compound represented by formula I is shown below:

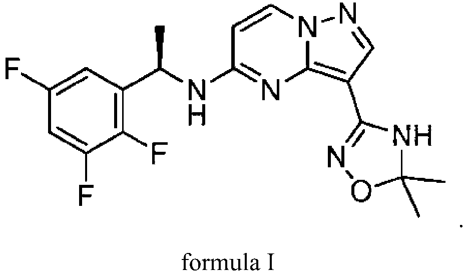

In one or more embodiments, the crystal form A has one or more characteristic peaks at 9.97±0.2°, 13.16±0.2°, 19.15±0.2°, 19.97±0.2° and 21.00±0.2° in the X-ray powder diffraction pattern expressed in terms of 2θ angles.

In one or more embodiments, the crystal form A has absorption peaks in X-ray powder diffraction expressed in terms of 2θ angles at the following positions:

| **Peak position [°2θ]** | **Relative intensity [%]** |
|---|---|
| 9.35±0.2° | 15.42 |
| 9.97±0.2° | 11.93 |
| 11.42±0.2° | 100.00 |
| 12.06±0.2° | 90.08 |
| 12.70±0.2° | 4.31 |
| 13.16±0.2° | 7.07 |
| 15.12±0.2° | 3.38 |
| 16.67±0.2° | 1.90 |
| 17.37±0.2° | 2.22 |
| 18.05±0.2° | 2.57 |
| 18.71±0.2° | 28.76 |
| 19.15±0.2° | 12.71 |
| 19.35±0.2° | 6.53 |
| 19.97±0.2° | 12.26 |
| 21.00±0.2° | 12.55 |
| 21.16±0.2° | 18.92 |
| 21.76±0.2° | 2.76 |
| 22.89±0.2° | 3.20 |
| 23.14±0.2° | 3.98 |
| 23.46±0.2° | 2.20 |
| 23.83±0.2° | 2.24 |
| 27.35±0.2° | 1.79 |
| 27.78±0.2° | 3.64 |
| 28.72±0.2° | 1.55 |
| 30.01±0.2° | 0.99 |

In one or more embodiments, the crystal form A has the X-ray powder diffraction pattern substantially as shown in FIG. 5.

In one or more embodiments, a thermogravimetric analysis (TGA) thermogram of the crystal form A shows 4.216% weight loss from room temperature (about 25°C) to 130°C.

In one or more embodiments, the crystal form A has a thermogravimetric analysis thermogram substantially as shown in FIG. 6.

In one or more embodiments, a differential scanning calorimetry (DSC) thermogram of the crystal form A shows endothermic peaks at about 87.11°C and 142.34°C.

In one or more embodiments, the crystal form A has a differential scanning calorimetry thermogram substantially as shown in FIG. 6.

In one or more embodiments, the crystal form A is a blocky crystal of 20-100 µm.

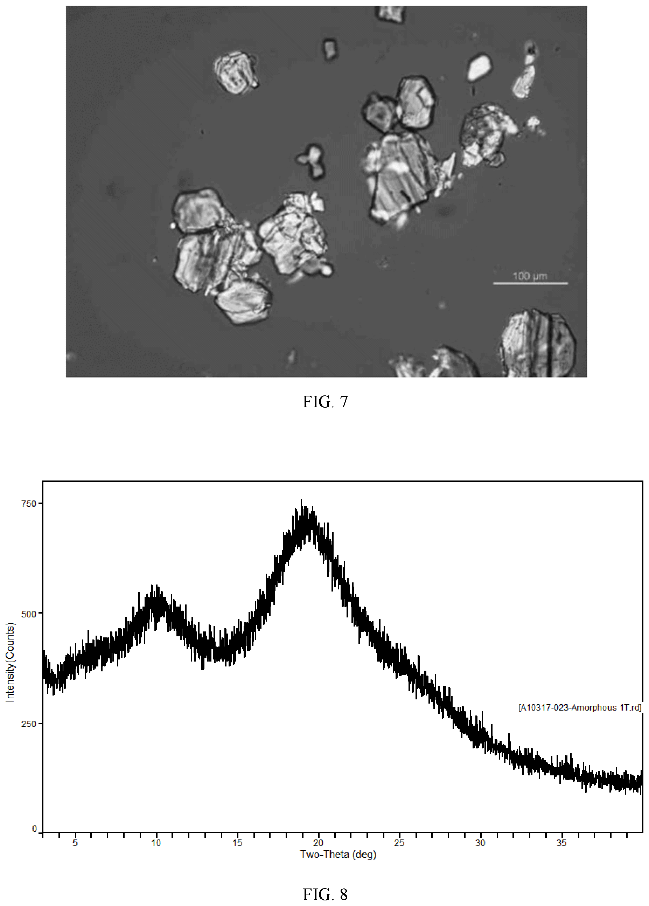

In one or more embodiments, the crystal form A has a morphology substantially as shown in FIG. 7.

In one or more embodiments, the crystal form A belongs to a monoclinic system and belongs to a space group P2(1), with unit cell parameters: a=10.2499(10) Å, b=17.9931(16) Å, c= 11.2407(8) Å, α=γ=90° β=93.349(8)°, deviation factor R1=0.0562, and Z=4.

One or more embodiments of the present disclosure provide a preparation method for the crystal form A, which comprises slurrying the compound represented by formula I using a mixed solvent of acetonitrile and water.

In one or more embodiments, a volume ratio of acetonitrile to water is 1: (1-3), such as 1: 1 or 1: 2.

In one or more embodiments, the slurrying is carried out at room temperature.

In one or more embodiments, the crystal form A is prepared by the following method: taking the compound represented by formula I, adding the mixed solvent of acetonitrile and water for a slurrying experiment, slurrying for 1-4 days, and then centrifuging to collect a crystalline powder solid.

One or more embodiments of the present disclosure provide an amorphous form of the compound represented by formula I, which has an X-ray powder diffraction pattern substantially as shown in FIG. 8.

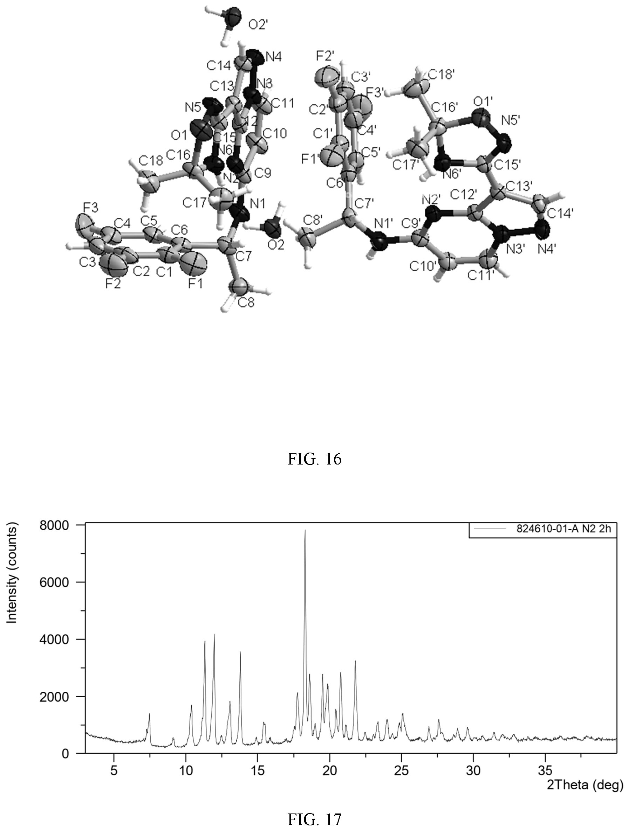

One or more embodiments of the present disclosure provide a crystal form B of the compound represented by formula I, which has an X-ray powder diffraction pattern substantially as shown in FIG. 17.

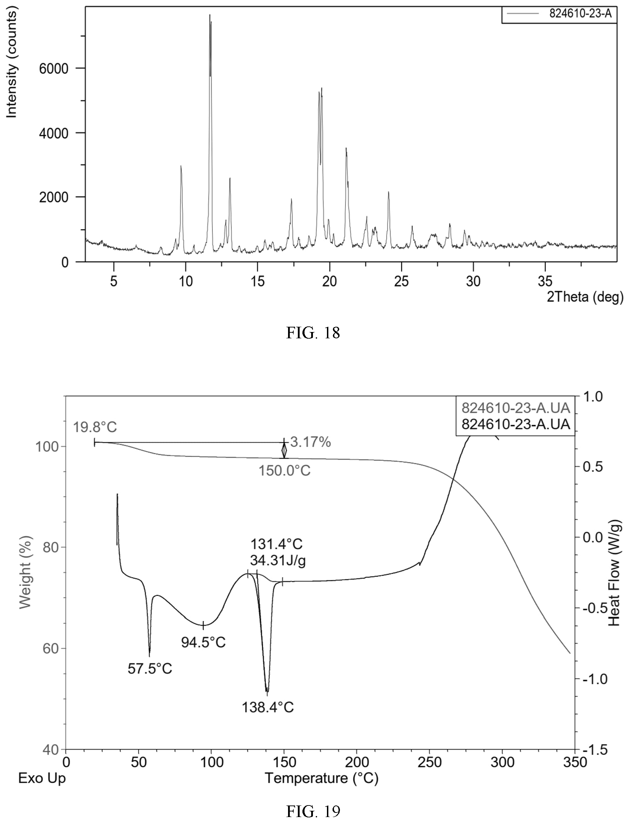

One or more embodiments of the present disclosure provide a crystal form C of the compound represented by formula I, which has an X-ray powder diffraction pattern substantially as shown in FIG. 18.

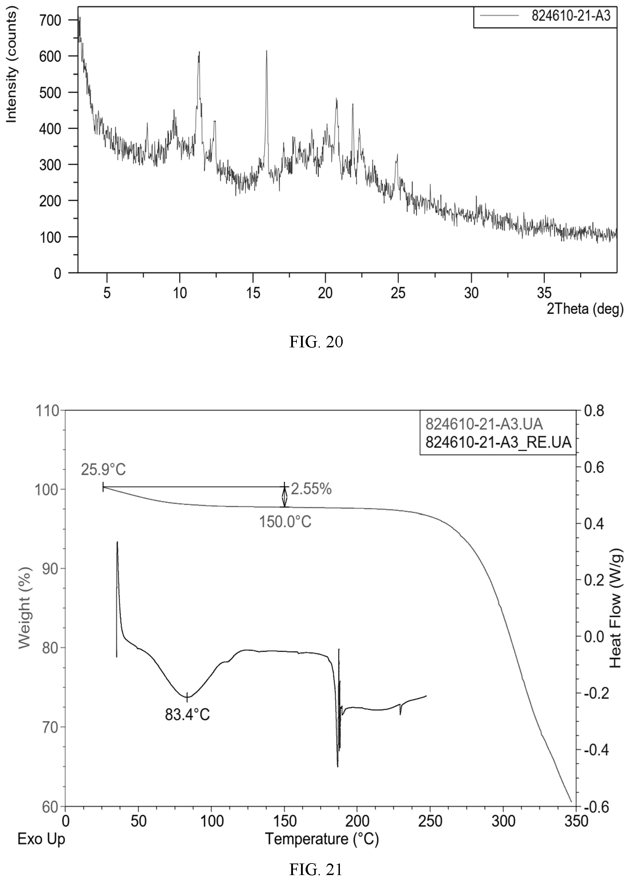

One or more embodiments of the present disclosure provide a crystal form D of the compound represented by formula I, which has an X-ray powder diffraction pattern substantially as shown in FIG. 20.

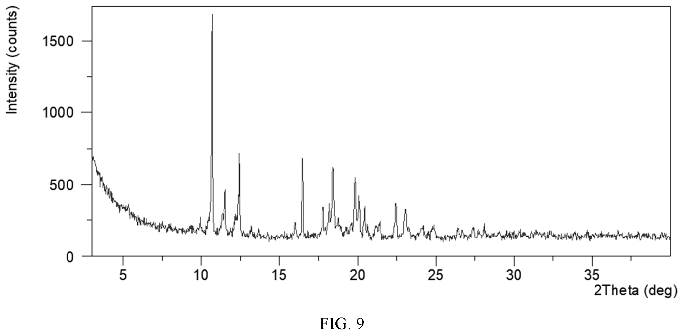

One or more embodiments of the present disclosure provide a crystal form E of the compound represented by formula I, which has an X-ray powder diffraction pattern substantially as shown in FIG. 9.

One or more embodiments of the present disclosure provide the use of the crystal form A of the compound represented by formula I in the preparation of a pharmaceutical composition.

One or more embodiments of the present disclosure provide the use of the crystal form A of the compound represented by formula I in the manufacture of a medicament for preventing and/or treating a disease with ROS1-, NTRK- or ALK-mediated pathological characteristics.

In one or more embodiments, the disease with ROS1-, NTRK- or ALK-mediated pathological characteristics comprises cancer, sarcoma and pain.

In one or more embodiments, the cancer is any one of a breast cancer, a cervical cancer, a colon cancer, a lung cancer, a gastric cancer, a rectal cancer, a pancreatic cancer, a brain cancer, a skin cancer, an oral cancer, a prostate cancer, a bone cancer, a renal cancer, an ovarian cancer, a bladder cancer, a liver cancer, a fallopian tube tumor, a peritoneal tumor, a melanoma, a glioma, a glioblastoma, a head and neck cancer, a papillary renal tumor, leukemia, a lymphoma, a myeloma and a thyroid tumor.

In one or more embodiments, the crystal form A of the compound represented by formula I is used for preventing and/or treating the following diseases: inflammation, cancer, cardiovascular diseases, infections, immune diseases and metabolic diseases.

One or more embodiments of the present disclosure provide a treatment method, which comprises the step of administering the crystal form A of the compound represented by formula I of the present disclosure to a subject in need thereof for selectively inhibiting fusion mutations and drug-resistant mutations of ROS1, NTRK, ALK, and the like.

In one or more embodiments, the crystal form A has very important significance for improving the production and quality control of drug, the development prospect of solid oral formulation, and other properties.

In one or more embodiments, results of physicochemical stability evaluation show that the crystal form A is a monohydrate, which does not lose water or have hygroscopicity under conventional conditions.

In one or more embodiments, a stability of the crystal form A is significantly higher than those of the amorphous form and the crystal form E under conditions of high temperature, high humidity, accelerated test and light exposure test.

In one or more embodiments, a flowability of the crystal form A is better than those of the crystal form E and the amorphous form.

In one or more embodiments, a bioavailability of the crystal form A is significantly higher than that of the amorphous form.

In one or more embodiments, due to good physicochemical stability, flowability and bioavailability, the crystal form A has great potential in subsequent development and production.

One or more embodiments of the present disclosure provide a composition of the present disclosure or a crystal form A of a compound represented by formula I for use in the treatment and/or prevention of a disease.

One or more embodiments of the present disclosure provide a composition of the present disclosure or a crystal form A of a compound represented by formula I, for use in the treatment and/or prevention of cancer or tumor.

One or more embodiments of the present disclosure provide a composition of the present disclosure or a crystal form A of a compound represented by formula I for use in the treatment and/or prevention of a breast cancer, a cervical cancer, a colon cancer, a lung cancer, a gastric cancer, a rectal cancer, a pancreatic cancer, a brain cancer, a skin cancer, an oral cancer, a prostate cancer, a bone cancer, a renal cancer, an ovarian cancer, a bladder cancer, a liver cancer, a fallopian tube tumor, a peritoneal tumor, a melanoma, a glioma, a glioblastoma, a head and neck cancer, a papillary renal tumor, leukemia, a lymphoma, a myeloma or a thyroid tumor.

One or more embodiments of the present disclosure provide a composition of the present disclosure or a crystal form A of a compound represented by formula I for use in the treatment and/or prevention of a disease mediated by ROS1, NTRK or ALK.

One or more embodiments of the present disclosure provide a method for treating and/or preventing a disease, which comprises administering the composition of the present disclosure or the crystal form A of the compound represented by formula I to a subject in need thereof.

One or more embodiments of the present disclosure provide a method for treating and/or preventing cancer or tumor, which comprises administering the composition of the present disclosure or the crystal form A of the compound represented by formula I to a subject in need thereof.

One or more embodiments of the present disclosure provide a method for treating and/or preventing breast cancer, cervical cancer, colon cancer, lung cancer, gastric cancer, rectal cancer, pancreatic cancer, brain cancer, skin cancer, oral cancer, prostate cancer, bone cancer, renal cancer, ovarian cancer, bladder cancer, liver cancer, fallopian tube tumor, peritoneal tumor, melanoma, glioma, glioblastoma, head and neck cancer, papillary renal tumor, leukemia, lymphoma, myeloma or thyroid tumor, which comprises administering the composition of the present disclosure or the crystal form A of the compound represented by formula I to a subject in need thereof.

One or more embodiments of the present disclosure provide a method for treating and/or preventing a disease mediated by ROS1, NTRK or ALK, which comprises administering the composition of the present disclosure or the crystal form A of the compound represented by formula I to a subject in need thereof.

It should be understood that, within the scope of the present disclosure, the above technical features of the present disclosure and the technical features specifically described hereinafter can be combined with each other to form new or preferred technical solutions. Due to space limitations, the technical solutions are not repeatedly described herein one by one.

### BRIEF DESCRIPTION OF THE DRAWINGS

FIG. 1 shows a dissolution curve of tablet 1 obtained in Composition Example 1.
FIG. 2 shows a dissolution curve of tablet 2 obtained in Composition Example 2.
FIG. 3 shows dissolution curves of tablets 3 and 4 obtained in Composition Examples 3 and 4.
FIG. 4 shows dissolution curves of tablets 5 and 6 obtained in Composition Examples 5 and 6.
FIG. 5 shows an XRD pattern of crystal form A.
FIG. 6 shows a thermogravimetric analysis and differential scanning calorimetry (TGA-DSC) thermogram of crystal form A.
FIG. 7 shows a polarized light micrograph (PLM) of crystal form A.
FIG. 8 shows an XRD pattern of an amorphous form.
FIG. 9 shows an XRD pattern of crystal form E.
FIG. 10 shows DVS test results of crystal form A.
FIG. 11 shows DVS test results of crystal form E.
FIG. 12 shows DVS test results of an amorphous form sample.
FIG. 13 shows flowability results of crystal form A, crystal form E and the amorphous form sample.
FIG. 14 shows the plasma concentration-time curves of a compound represented by formula I (crystal form A) after administration to rats at a dose of 10 mg/kg.
FIG. 15 shows the plasma concentration-time curves of a compound represented by formula I (amorphous form) after administration to rats at a dose of 10 mg/kg.
FIG. 16 shows a structural diagram of a single crystal of crystal form A of the compound represented by formula I (FIG. 16 shows a asymmetric unit, containing 2 molecules of the compound and 1 molecule of crystal water, wherein both C7 and C7' are in R configuration, with the FLACK parameter is 0.26(12)).
FIG. 17 shows an XRD pattern of crystal form B.
FIG. 18 shows an XRD pattern of crystal form C.
FIG. 19 shows a thermogravimetric analysis and differential scanning calorimetry (TGA-DSC) thermogram of crystal form C.
FIG. 20 shows an XRD pattern of crystal form D.
FIG. 21 shows a thermogravimetric analysis and differential scanning calorimetry (TGA-DSC) thermogram of crystal form D.
FIG. 22 shows an XRD pattern of crystal form D after standing sealed at room temperature for 2 days.
FIG. 23 shows an XRD pattern of crystal form D after heating to 120°C and cooling to room temperature.
FIG. 24 and FIG. 25 show XRD patterns of crystal forms A/C/D after suspension competition at different water activities at room temperature.

### DETAILED DESCRIPTION

The technical solutions of the present disclosure will be further described in details hereinafter with reference to specific embodiments. It should be understood that the following embodiments are merely for the purposes of illustrating and explaining the present disclosure, and should not be construed as limiting the scope of protection of the present disclosure. Any technology implemented based on the above contents of the present disclosure falls within the intended scope of protection of the present disclosure.

Unless otherwise specified, the raw materials and reagents used in the following examples are all commercially available products or can be prepared by known methods.

After long-term and in-depth research, the inventors have obtained a pharmaceutical composition with excellent dissolution properties, pharmacokinetic properties and storage stability by composition optimization. On this basis, the inventors have completed the present disclosure.

Following oral administration, the drug can be absorbed at multiple different sites along the gastrointestinal tract (including via the stomach, duodenum, jejunum, ileum, and colon). There is a significant pH change between the stomach (pH 1-3.5) and the small intestine (pH 4-8), resulting in different pH conditions at each absorption site. Studies conducted in the present application have found that the solubility of TY-2136b is pH-dependent, increasing with rising pH, but the solubility is generally low. In dissolution studies, it was observed that the sink condition can be satisfied in pH 6.8 medium supplemented with SDS.

One or more embodiments of the present disclosure provide a pharmaceutical composition suitable for oral administration, and more particularly relate to a pharmaceutical composition (such as a pharmaceutical tablet) comprising TY-2136b or a pharmaceutically acceptable hydrate, solvate or salt thereof. Meanwhile, the solid dosage form of the composition of the present disclosure shows excellent storage stability.

In one or more embodiments, the pharmaceutical composition of the present disclosure may be formed into a tablet, which shows improved dissolution characteristics under physiologically relevant conditions and/or a higher total release of a drug thereof in terms of physiologically relevant indicators.

Generally, crystallization may lead to formation of a solvate of a compound of the present disclosure. As used herein, the term "solvate" refers to an aggregate including one or more molecules of TY-2136b and one or more solvent molecules. The solvent may be water, and in this case, the solvate may be a hydrate. Optionally, the solvent may be an organic solvent. Therefore, the compound of the present disclosure may exist as a hydrate, comprising a monohydrate, a dihydrate, a hemihydrate, a sesquihydrate, a trihydrate, a tetrahydrate, and the like, and as a corresponding solvated form.

"Pharmaceutical composition" refers to a formulation of the compound of the present disclosure with a medium generally accepted in the art for delivering a biologically active compound to a mammal, such as a human. Such media comprises all pharmaceutically acceptable carriers, diluents or excipients.

"Pharmaceutically acceptable carrier, diluent or excipient" comprises, but is not limited to, any acceptable adjuvant, carrier, excipient, glidant, sweetener, diluent, preservative, dye/colorant, flavoring agent, surfactant, wetting agent, dispersing agent, suspending agent, stabilizer, isotonic agent, solvent or emulsifier approved by the U.S. Food and Drug Administration for use in humans or domestic animals.

As used herein, the term "pharmaceutically acceptable salt" refers to salts of the compounds of the present disclosure formed with acids or bases that are suitable for use as pharmaceuticals. Pharmaceutically acceptable salts comprise inorganic salts and organic salts. One class of preferred salts are salts formed from the compounds of the present disclosure with acids. Suitable acids for forming such salts comprise, but are not limited to: inorganic acids such as hydrochloric acid, hydrobromic acid, hydrofluoric acid, sulfuric acid, nitric acid, phosphoric acid; organic acids such as formic acid, acetic acid, trifluoroacetic acid, propionic acid, oxalic acid, malonic acid, succinic acid, fumaric acid, maleic acid, lactic acid, malic acid, tartaric acid, citric acid, picric acid, benzoic acid, methanesulfonic acid, ethanesulfonic acid, p-toluenesulfonic acid, benzenesulfonic acid, naphthalenesulfonic acid; and amino acids such as proline, phenylalanine, aspartic acid, glutamic acid.

Another class of preferred salts are salts formed from the compounds of the present disclosure with bases, such as alkali metal salts (e.g., sodium or potassium salts), alkaline earth metal salts (e.g., magnesium or calcium salts), ammonium salts (including lower alkylolammonium salts and other pharmaceutically acceptable amine salts), for example, methylamine salts, ethylamine salts, propylamine salts, dimethylamine salts, trimethylamine salts, diethylamine salts, triethylamine salts, tert-butylamine salts, ethylenediamine salts, ethanolamine salts, diethanolamine salts, triethanolamine salts, and amine salts formed from morpholine, piperazine, and lysine, respectively.

Because TY-2136b shows excellent anti-tumor activity, the pharmaceutical composition comprising TY-2136b as a main active ingredient may be used for treating, preventing and alleviating a tumor-related disease.

The pharmaceutical composition of the present disclosure comprises a safe and effective amount of the compound of the present disclosure or a pharmacologically acceptable salt thereof, and a pharmacologically acceptable excipient or carrier. The "safe and effective amount" used herein means an amount of the compound sufficient to significantly ameliorate the condition without causing severe side effects. Generally, the pharmaceutical composition contains 1-2000 mg of the compound of the present disclosure per dose, and more preferably 10-1000 mg of the compound of the present disclosure per dose. Preferably, the "one dose" refers to one capsule or tablet.

"Pharmaceutically acceptable carrier" refers to one or more compatible solid or liquid fillers or gel substances which are suitable for human use and must be of sufficient purity and sufficiently low toxicity. "Compatibility" as used herein means that the components of the composition can be blended with the compounds of the present disclosure and with each other without significantly reducing the pharmacological activity of the compounds. Examples of pharmaceutically acceptable carrier comprise cellulose and its derivatives (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyhydric alcohols (such as propylene glycol, glycerol, mannitol, sorbitol, etc.), emulsifiers (such as Tween^{®}), wetting agents (such as sodium dodecyl sulfate), colorants, flavoring agents, stabilizers, antioxidants, preservatives, and pyrogen-free water.

The pharmaceutical composition of the present application is in the form of an injection, capsule, tablet, pill, powder, or granule.

There is no particular limitation on the administration mode of the compounds of the present invention or the pharmaceutical compositions. Representative administration modes comprise (but are not limited to): oral administration, intratumoral administration, rectal administration, parenteral administration (intravenous, intramuscular or subcutaneous), and topical administration.

Solid dosage forms for oral administration comprise capsules, tablets, pills, powders, and granules. In these solid dosage forms, the active compound is mixed with at least one conventional inert excipient (or carrier), such as sodium citrate or dicalcium phosphate, or with the following ingredients: (a) fillers or extenders, for example, starch, lactose, sucrose, glucose, mannitol, and silicic acid; (b) binders, for example, hydroxymethyl cellulose, alginates, gelatin, polyvinylpyrrolidone, sucrose, and acacia; (c) humectants, for example, glycerol; (d) disintegrants, for example, agar, calcium carbonate, potato starch or tapioca starch, alginic acid, certain composite silicates, and sodium carbonate; (e) buffering agents, for example, paraffin; (f) absorption accelerators, for example, quaternary ammonium compounds; (g) wetting agents, for example, cetyl alcohol and glyceryl monostearate; (h) adsorbents, for example, kaolin; and (i) lubricants, for example, talc, calcium stearate, magnesium stearate, solid polyethylene glycols, sodium dodecyl sulfate, or mixtures thereof. In capsules, tablets, and pills, the dosage forms may also contain buffering agents.

Solid dosage forms such as tablets, dragees, capsules, pills, and granules can be prepared with coatings and shells, such as enteric coatings and other materials well known in the art. They may contain opacifying agents, and the release of the active compound or compounds in such compositions can be delayed in a certain part of the digestive tract. Examples of suitable embedding components are polymeric substances and waxy substances. If necessary, the active compound can also be formulated into microcapsules with one or more of the above-mentioned excipients.

Liquid dosage forms for oral administration comprise pharmaceutically acceptable emulsions, solutions, suspensions, syrups and tinctures. In addition to the active compound, the liquid dosage forms may contain an inert diluents conventionally used in the art, such as water or other solvents, solubilizers and an emulsifiers, for example, ethanol, isopropanol, ethyl carbonate, ethyl acetate, propylene glycol, 1,3-butanediol, dimethylformamide and oils, especially cottonseed oil, peanut oil, corn oil, olive oil, castor oil and sesame oil, or mixtures of these substances.

In addition to these inert diluents, the composition may also contain an adjuvant, such as a wetting agent, an emulsifying agent and a suspending agent, a sweetening agent, a flavoring agent and a perfume.

In addition to the active compound, suspension may contain a suspending agent, for example, ethoxylated isostearyl alcohol, polyoxyethylene sorbitol and sorbitan ester, microcrystalline cellulose, aluminum methoxide and agar, or a mixture of these substances.

The composition for parenteral injection may include a physiologically acceptable sterile aqueous or non-aqueous solution, dispersion, suspension or emulsion, and a sterile powder for reconstitution into sterile injectable solution or dispersion. Suitable aqueous and non-aqueous carriers, diluents, solvents or excipients comprise water, ethanol, a polyol and a suitable mixture of these substances.

Dosage forms of the compound of the present disclosure for topical administration comprise ointments, powders, patches, sprays and inhalants. The active ingredient is mixed under sterile conditions with a physiologically acceptable carrier and any preservatives and buffers, or a propellants that may be required if necessary.

The composition of the present disclosure may be administered alone or in combination with other pharmaceutically acceptable compositions (such as an anti-tumor drug).

The treatment method of the present disclosure may be applied alone or in combination with other therapeutic means or therapeutic drugs.

When the pharmaceutical composition is applied, a safe and effective amount of the compound of the present disclosure is administered to a mammal (such as a human) in need of treatment, wherein the dosage for administration is a pharmaceutically effective dose. For a human weighing 60 kg, the daily dosage is generally 1-2000 mg, preferably 50-1000 mg. The specific dosage should also take into account factors such as the route of administration, the patientt's health condition, etc., , all of which are within the skill of a skilled physician.

The specific examples of the present disclosure specifically describe the preparation methods of the pharmaceutical composition of the present disclosure, but these specific methods do not constitute any limitation to the present disclosure. The pharmaceutical composition of the present disclosure can also be conveniently prepared by optionally combining various preparation methods described in the present specification or known in the art, and such combinations can be readily performed by those skilled in the art to which the present disclosure belongs.

Typically, the raw materials and reagents used in the preparation process of the pharmaceutical composition of the present disclosure are all commercially available unless otherwise specified.

In one or more embodiments, the pharmaceutical composition of the present disclosure has one or more of the following beneficial effects:
(1) the pharmaceutical composition has excellent dissolution properties, pharmacokinetic properties and storage stability;
(2) the pharmaceutical composition is stable in quality;
(3) the preparation process of the pharmaceutical composition is simple and feasible, thereby facilitating connection with industrial production; and
(4) the pharmaceutical composition is convenient for administration, safe and reliable for use, well accepted by patients, and has high social and economic value.

### EMBODIMENTS

The present disclosure is further explained hereinafter with reference to specific embodiments. It should be understood that these embodiments are only used for explaining the present disclosure and are not intended to limit the scope of the present disclosure. Experimental methods for which specific conditions are not indicated in the following embodiments are generally carried out under conventional conditions, such as those described in Molecular Cloning: A Laboratory Manual of Sambrook et al. (New York: Cold Spring Harbor Laboratory Press, 1989), or under conditions recommended by the manufacturers. Unless otherwise stated, percentages and parts are calculated by weight.

Unless otherwise defined, all professional and scientific terms used herein have the same meanings well known to those skilled in the art. In addition, any methods and materials similar or equivalent to those described herein may be used in the method of the present disclosure. The preferred implementing methods and materials described herein are for illustrative purposes only.

### Composition-related examples

### General raw materials

| Name of material | Model |
|---|---|
| TY-2136b | N/A |
| Lactose | Tablettose80 |
| Microcrystalline cellulose | 102 |
| Colloidal silicon dioxide | 200 |

| Magnesium stearate | LIGAMED MF-2-V |
|---|---|
| Film coating premix (gastric-soluble) | Opadry^{®} 85F620077 |

### General method

### Dissolution performance

Dissolution is defined as the rate and extent to which a drug dissolves from a solid dosage form such as a tablet in a specified solvent. Dissolution is a critical quality attribute for tablet quality control, and dissolution testing is routinely required for poorly soluble drugs. The dissolution test procedure comprises placing a specified quantity of a solid dosage form in the basket (or dissolution vessel) of a dissolution apparatus, operating at a constant temperature of 37°C ± 0.5°C, a specified rotational speed, and in a specified dissolution medium, sampling at predetermined time points, and determining the amount of drug dissolved.

Dissolution test conditions are as follows:
dissolution medium: pH 6.8 phosphate buffer+0.2% SDS
volume of dissolution medium : 900 mL
rotation speed: 50 rpm (final rotation speed: 250 rpm at 60-75 minutes)
temperature of water bath: 37±0.5°C
sampling time points: 5, 15, 30, 45, 60, 75 minutes

### Composition Example 1 Tablet 1 and preparation thereof (formulation composition (40 mg))

| **Names of raw and auxiliary materials** | **Proportion% (w/w)** | **mg/tablet** | **Function** |
|---|---|---|---|
| TY-2136b (D90 17.00 µm) | 10.46 | 41.85 | Drug substance |
| Lactose | 39.77 | 159.08 | Diluent |
| Microcrystalline cellulose | 39.77 | 159.08 | Diluent |
| Colloidal silicon dioxide | 2.00 | 8.00 | Glidant |
| Croscarmellose sodium | 7.00 | 28.00 | Disintegrant |
| Magnesium stearate (intragranular) | 0.50 | 2.00 | Lubricant |
| Magnesium stearate (extragranular) | 0.50 | 2.00 | Lubricant |
| **Total uncoated tablet** | 100.00 | 400.00 | |
| Film coating premix | 3.00 | 12.00 | Coating |
| (gastric-soluble) | | | |
| **Total coated tablet** | 103.00 | 412.00 | |

Note: the TY-2136b used contained one molecule of water, and 41.85 mg of hydrate was equivalent to 40 mg of TY-2136b free base.

Tablet 1 was manufactured by a dry blending/compression compaction process with the materials listed in the table above. TY-2136b, colloidal silicon dioxide, croscarmellose sodium and microcrystalline cellulose were each sieved through a 40-mesh screen, then added sequentially into a blending hopper, and mixed at a rotation speed of 20 rpm for 25 minutes. Magnesium stearate (intragranular addition) was then added into the bleding hopper, and mixed at a rotation speed of 20 rpm for 5 minutes to obtain a premix. The premix was added into a dry granulator for dry granulation, under a rotary feeding with a rotation speed of 20-30 rpm, a forming pressure of 25-35 bar (specifically 30 bar), a roller rotation speed of 10-20 rpm (specifically 15 rpm) and a sizing rotation speed of 120-180 rpm (specifically 150 rpm), so as to obtain dry-granulated granules. The granules collected after dry granulation and the sieved magnesium stearate (extragranular addition) were added into the blending hopper, and mixed at a rotation speed of 20 rpm for 5 minutes (which was a final blending time). The mixture was compressed into an uncoated tablet 1 by using punches (hardness 81-115 N).

The film coating premix (12 mg/tablet) was prepared into a 12% (w/w) coating suspension, and the uncoated tablet 1 was coated according to 3% of the weight of the uncoated tablet, so as to form tablet 1.

Dissolution results of the obtained tablet 1 can be seen in Table 1 below.

**Table 1**

| Time (min) | 0 | 5 | 15 | 30 | 45 | 60 | 75 |
|---|---|---|---|---|---|---|---|
| Dissolution (%) | 0 | 41 | 83 | 93 | 97 | 99 | 102 |

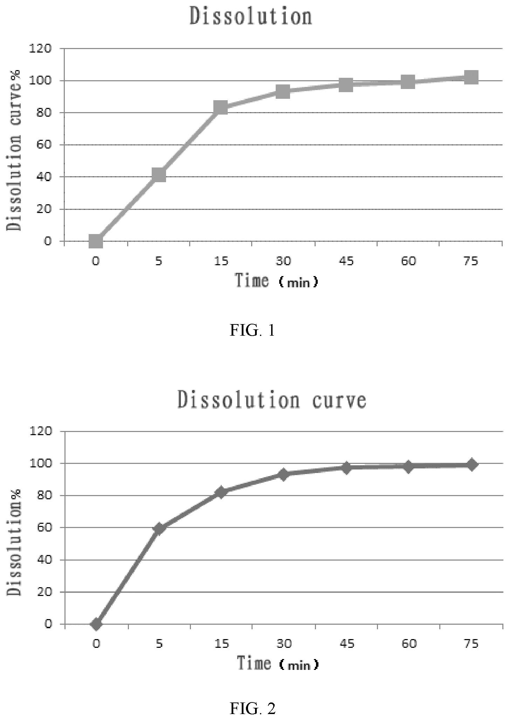

FIG. 1 shows a dissolution curve of tablet 1 obtained in Example 1.

As shown in FIG. 1 and Table 1 above, tablet 1 could be completely dissolved under the above dissolution conditions, and the dissolution at 45 minutes had reached 97% of a labeled amount, which met the standard.

### Composition Example 2 Tablet 2 and preparation thereof (formulation composition (10 mg))

| **Names of raw and auxiliary materials** | **Proportion% (w/w)** | **mg/tablet** | **Function** |
|---|---|---|---|
| TY-2136b (D90 17.00 µm) | 10.46 | 10.46 | Drug substance |
| Lactose | 39.77 | 39.77 | Diluent |
| Microcrystalline cellulose | 39.77 | 39.77 | Diluent |
| Colloidal silicon dioxide | 2.00 | 2.00 | Glidant |
| Croscarmellose sodium | 7.00 | 7.00 | Disintegrant |
| Magnesium stearate (intragranular) | 0.50 | 0.50 | Lubricant |
| Magnesium stearate (extragranular) | 0.50 | 0.50 | Lubricant |
| Total uncoated tablet | 100.00 | 100.00 | |
| Film coating premix (gastric-soluble) | 3.00 | 3.00 | Coating |
| Total coated tablet | 103.00 | 103 | |

Note: the TY-2136b used contained one molecule of water, and 10.46 mg of hydrate was equivalent to 10 mg of TY-2136b free base.

Tablet 2 was manufactured by a dry blending/compression process with the materials listed in the table above. TY-2136b, colloidal silicon dioxide, croscarmellose sodium and microcrystalline cellulose were each sieved through a 40-mesh screen, then added sequentially into a blending hopper, and mixed at a rotation speed of 20 rpm for 25 minutes. Magnesium stearate (intragranular addition) was added into the blending hopper, and mixed at a rotation speed of 20 rpm for 5 minutes to obtain a premix. The premix was added into a dry granulator for dry granulation, under a rotary feeding with a rotation speed of 20-30 rpm, a forming pressure of 25-35 bar (specifically 30 bar), a roller rotation speed of 10-20 rpm (specifically 15 rpm) and a sizing rotation speed of 120-180 rpm (specifically 150 rpm), so as to obtain dry-granulated granules. The granules collected after dry granulation and the sieved magnesium stearate (extragranular addition) were added into the blending hopper, and mixed at a rotation speed of 20 rpm for 5 minutes (which was a final blending time). The mixture was compressed into an uncoated tablet 2 by using punches (hardness 53-78 N).

The film coating premix (3 mg/tablet) was prepared into a 12% (w/w) coating suspension, and the uncoated tablet 2 was coated according to 3% of weight of the uncoated tablet, so as to form tablet 2.

**Table 2**

| Time (min) | 0 | 5 | 15 | 30 | 45 | 60 | 75 |
|---|---|---|---|---|---|---|---|
| Dissolution (%) | 0 | 59 | 82 | 93 | 97 | 98 | 99 |

FIG. 2 shows a dissolution curve of tablet 2 obtained in Example 2.

As shown in FIG. 2 and Table 2 above, tablet 2 could be completely dissolved under the above dissolution conditions, and the dissolution at 45 minutes had reached 97% of a labeled amount, which met the standard.

### Composition Examples 3-4 Tablets 3 and 4

These examples were the same as Example 1, with a main difference in an amount of disintegrant, as specifically shown in Table 3 below, and dissolution results can be seen in Table 4 below.

**Table 3 Formulation compositions with different amounts of disintegrant**

| | Example 3 | | Example 4 | |
|---|---|---|---|---|
| **Names of raw and auxiliary materials** | **Proportion % (w/w)** | mg/tablet | **Proportion % (w/w)** | mg/tablet |
| TY-2136b (D90 17µm) | 10.46 | 41.85 | 10.46 | 41.85 |
| Lactose | 41.77 | 167.08 | 40.77 | 163.08 |
| Microcrystalline cellulose | 41.77 | 167.08 | 40.77 | 163.08 |
| Colloidal silicon dioxide | 2.00 | 8.00 | 2.00 | 8.00 |
| Croscarmellose sodium | 3.00 | 12.00 | 5.00 | 20.00 |
| Magnesium stearate (intragranular) | 0.50 | 2.00 | 0.50 | 2.00 |
| Magnesium stearate (extragranular) | 0.50 | 2.00 | 0.50 | 2.00 |
| Total uncoated tablet | 100.00 | 400.00 | 100.00 | 400.00 |
| Film coating premix (gastric-soluble) | 3.00 | 12.00 | 3.00 | 12.00 |
| Total coated tablet | 103.00 | 412.00 | 103.00 | 412.00 |

**Table 4 Effects of different amounts of disintegrant on dissolution rate**

| | Amount of disintegrant | pH 6.8+0.5% SDS, paddle method 50 rpm, 900 mL | | | | | |
|---|---|---|---|---|---|---|---|
| | | Dissolution (%, n=3) | | | | | |
| | | 5 minutes | 15 minutes | 30 minutes | 45 minutes | 60 minutes | 75 minutes |
| Example 3 | 3% | 38 | 57 | 68 | 72 | 75 | 93 |
| Example 4 | 5% | 51 | 69 | 79 | 84 | 86 | 99 |

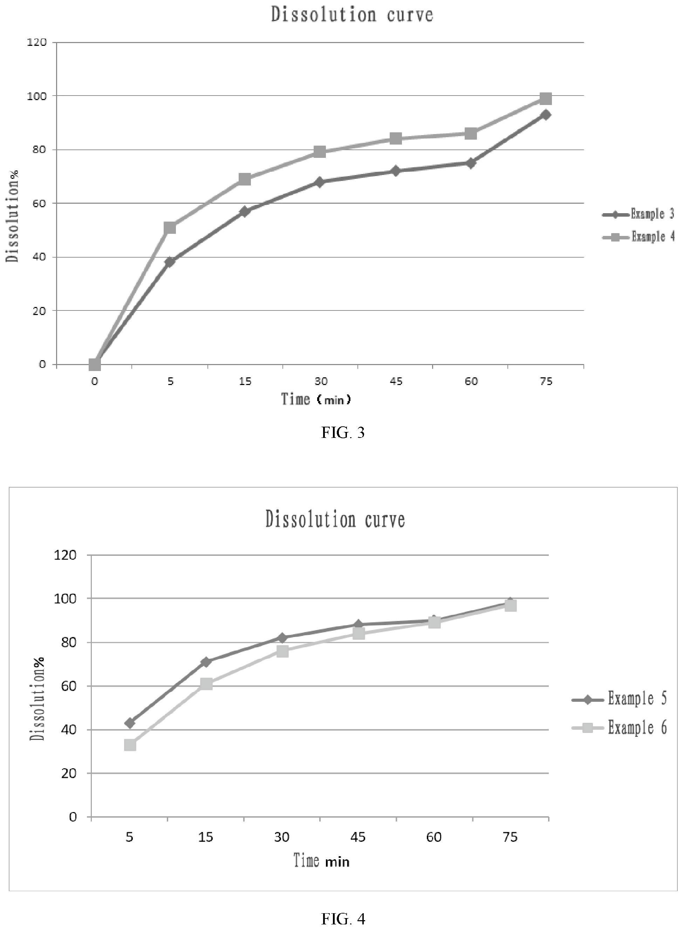

As shown in FIG. 3 and Table 4 above, tablet 4 could be completely dissolved under the above dissolution conditions, and the dissolution at 45 minutes had reached 84% of a labeled amount, which met the standard.

### Composition Examples 5-6 Tablets 5 -6

These examples were the same as Example 3, with differences in a different particle size of TY-2136b in the formulation and the use of direct powder compression in the process, as specifically shown in Table 5 below, and dissolutions can be seen in Table 6 below.

**Table 5 Formulation compositions with different particle sizes of API**

| | **Example 5** | | **Example 6** | |
|---|---|---|---|---|
| **Process** | **Direct powder compression** | | **Direct powder compression** | |
| **Particle size distribution of active pharmaceutical ingredient** | **D90 124 µm** | | **D90 191 µm** | |
| **Component** | **w/w %** | **mg/tablet** | **w/w %** | **mg/tablet** |
| TY-2136b | 10.46 | 41.85 | 10.46 | 41.85 |
| Lactose | 42.77 | 171.08 | 42.77 | 171.08 |
| Microcrystalline cellulose 102 | 42.77 | 171.08 | 42.77 | 171.08 |
| Croscarmellose sodium | 3.00 | 12.00 | 3.00 | 12.00 |
| Magnesium stearate | 1.00 | 4.00 | 1.00 | 4.00 |
| Total | 100.00 | 400.00 | 100.00 | 400.00 |
| Film coating premix (gastric-soluble) | 3.00 | 12.00 | 3.00 | 12.00 |
| Total coated tablet | 103.00 | 412.00 | 103.00 | 412.00 |

**Table 6 Effects of different particle sizes of API on dissolution rate**

| **Formulation No.** | **API PSD** | **pH 6.8+0.2% SDS, paddle method, 50 rpm, 900 mL** | | | | | |
|---|---|---|---|---|---|---|---|
| | | **Dissolution (%, n=3)** | | | | | |
| | | **5 minutes** | **15 minutes** | **30 minutes** | **45 minutes** | **60 minutes** | **75 minutes** |
| Example 5 | D90 124 µm | 43 | 71 | 82 | 88 | 90 | 98 |
| Example 6 | D90 191 µm | 33 | 61 | 76 | 84 | 89 | 97 |

As shown in FIG. 4 and Table 6 above, tablets 5 and 6 could be completely dissolved under the above dissolution conditions, and the dissolution at 45 minutes had reached above 80% of a labeled amount, which met the standard.

### Composition Example 7

The inventors produced tablet 1 (40 mg) and the tablet 2 (10 mg) according to the formulations in Example 1 and Example 2. The above samples were stored under conditions of 40°C/75%RH (open), 40°C/75%RH (sealed), and 40°C/75%RH (sealed+1 g desiccant) to investigate dissolutions and related substances. Results can be seen in Table 7 and Table 8 below.

Table 7 shows dissolution results of the samples for stability study.

**Table 7**

| **Batch No.** | **Condition** | | **Dissolution (%, n=3)** | | | | | |
|---|---|---|---|---|---|---|---|---|
| | | | **5 minutes** | **15 minutes** | **30 minutes** | **45 minutes** | **60 minutes** | **75 minutes** |
| Tablet 2 (10 mg) | 0 day | | 59 | 82 | 93 | 97 | 98 | 99 |
| | 40°C/75%RH (open) | 10 days | 62 | 82 | 92 | 96 | 97 | 99 |
| | | 30 days | 65 | 86 | 95 | 98 | 99 | 101 |
| | 40°C/75%RH (sealed+1 g desiccant) | 10 days | 66 | 88 | 97 | 101 | 104 | 106 |
| | | 30 days | 67 | 86 | 95 | 98 | 99 | 100 |
| Tablet 1 (40 mg) | 0 day | | 41 | 83 | 93 | 97 | 99 | 102 |
| | 40°C/75%RH (open) | 10 days | 66 | 83 | 92 | 96 | 97 | 99 |
| | | 30 days | 65 | 84 | 93 | 98 | 100 | 101 |
| | 40°C/75%RH (sealed+1 g desiccant) | 10 days | 64 | 85 | 94 | 98 | 99 | 100 |
| | | 30 days | 66 | 84 | 93 | 96 | 97 | 99 |

Table 8 shows related substance results of the samples for stability study.

**Table 8**

| **Specification** | **Condition** | **Time** | **Impurities (%)** | | | | | | | | |
|---|---|---|---|---|---|---|---|---|---|---|---|
| | | | **Individual impurity (RRT)** | | | | | | | | **Total impurities** |
| | | | **0.56** | **0.66/ 0.67/ 0.69/ 0.70** | **0.71/ 0.72/ 0.73/ 0.74** | **0.91** | **1.02** | **1.05/ 1.06** | **1.07/ 1.08** | **1.14** | |
| Active pharmaceutical | N/A | | <0.05 | 0.22 | 0.18 | <0.05 | 0.27 | 0.07 | 0.18 | <0.05 | 0.92 |
| ingredient^{*1} | | | | | | | | | | | |
| Active pharmaceutical ingredient^{*2} | N/A | | <0.05 | 0.22 | 0.19 | <0.05 | 0.32 | 0.07 | 0.19 | <0.05 | 0.99 |
| Tablet 2 (10 mg) | TO | | <0.05 | 0.21 | 0.19 | <0.05 | 0.29 | 0.09 | 0.19 | 0.06 | 1.03 |
| | 40°C/75%RH (open) | 10 days | <0.05 | 0.22 | 0.23 | <0.05 | 0.26 | <0.05 | 0.18 | <0.05 | 0.89 |
| | | 30 days | <0.05 | 0.23 | 0.25 | <0.05 | 0.17 | ND | 0.25 | <0.05 | 0.90 |
| | 40°C/75%RH (sealed) | 10 days | <0.05 | 0.22 | 0.21 | <0.05 | 0.25 | 0.08 | 0.18 | <0.05 | 0.94 |
| | | 30 days | <0.05 | 0.22 | 0.21 | <0.05 | 0.17 | 0.05 | 0.24 | <0.05 | 0.89 |
| | 40°C/75%RH (sealed+1 g desiccant) | 10 days | <0.05 | 0.21 | 0.19 | <0.05 | 0.18 | 0.05 | 0.20 | <0.05 | 0.83 |
| | | 30 days | <0.05 | 0.23 | 0.20 | <0.05 | 0.35 | 0.06 | 0.21 | <0.05 | 1.05 |
| Tablet 1 (40 mg) | TO | | <0.05 | 0.22 | 0.18 | <0.05 | 0.27 | 0.08 | 0.19 | <0.05 | 0.94 |
| | 40°C/75%RH (open) | 10 days | <0.05 | 0.22 | 0.24 | <0.05 | 0.24 | <0.05 | 0.18 | <0.05 | 0.88 |
| | | 30 days | <0.05 | 0.22 | 0.25 | <0.05 | 0.16 | ND | 0.24 | <0.05 | 0.87 |
| | 40°C/75%RH (sealed) | 10 days | <0.05 | 0.21 | 0.20 | <0.05 | 0.19 | 0.07 | 0.17 | <0.05 | 0.84 |
| | | 30 days | <0.05 | 0.21 | 0.20 | <0.05 | 0.17 | 0.05 | 0.23 | <0.05 | 0.86 |
| | 40°C/75%RH (sealed+1 g | 10 days | <0.05 | 0.21 | 0.20 | <0.05 | 0.17 | 0.05 | 0.21 | <0.05 | 0.84 |
| | desiccant) | 30 days | <0.05 | 0.22 | 0.21 | <0.05 | 0.35 | 0.05 | 0.21 | <0.05 | 1.04 |

| | | | | | | | | | | | |
|---|---|---|---|---|---|---|---|---|---|---|---|
| Note: **1. The results of the active pharmaceutical ingredient in this row were issued in the same sequence as those of the two specifications T0 at 40°C*/*75 %RH (open) for 10 days and 40°C*/*75 %RH (closed) for 10 days.* **2. The results of the active pharmaceutical ingredient in this row were issued in the same sequence as those of the two specifications at 40 °C*/*75 %RH (closed+1 g desiccant) for 30 days.* | | | | | | | | | | | |

It can be seen from Table 7 that, after storage at 40°C/75 %RH (open) and 40°C/75 %RH (sealed+1 g desiccant) for 30 days, the tablet 1 (40 mg) and the tablet 2 (10 mg) had no significant changes in dissolution rate, which complied with the Chinese Pharmacopoeia.

It can be seen from Table 8 that, after storage at 40°C/75 %RH (open), 40°C/75 %RH (sealed) and 40°C/75 %RH (sealed+1 g desiccant) for 30 days, the individual impurities of tablet 1 (40 mg) and tablet 2 (10 mg) fluctuated within acceptable ranges.

### Crystal form-related examples

### Crystal Form Example 1 Preparation of crystal form A

15.3 mg of the compound represented by formula I was placed in a 1.5 mL HPLC vial, and 0.2 mL of ACN/H₂O (1: 1, v: v) was added for carrying out a slurry experiment at room temperature. After slurrying for approximately 4 days, a crystalline powder solid was collected by centrifugation, which was crystal form A. An XRD pattern of the crystal form A was shown in FIG. 5. A thermogravimetric analysis and differential scanning calorimetry (TGA-DSC) thermogram of the crystal form A was shown in FIG. 6. A polarized light micrograph (PLM) of the crystal form A was shown in FIG. 7.

**Table 9 Parameters of XRPD test**

| Instrument model | **X' Pert3 (**transmission**)** | **Empyrean (**reflection**)** |
|---|---|---|
| X ray | Cu, kα; Kα1 (Å: 1.540598, | Cu, kα; Kα1 (Å: 1.540598, |

| | Kα2 (Å: 1.544426 Intensity ratio Kα2/Kα1: 0.50 | Kα2 (Å: 1.544426 Intensity ratio Kα2/Kα1: 0.50 |
|---|---|---|
| Setting of X-ray tube | 45 kV, 40 mA | 45 kV, 40 mA |
| Divergence slit | 1/2° | Automatic |
| Scanning mode | Continuous | Continuous |
| Scanning range (°2θ) | 3-40 | 3-40 |
| Scanning step size (°2θ) | 0.0131 | 0.0263 |
| Scanning time per step (s) | 33.15 | 49.73 |
| Scanning time (s) | 7 min 03 s | 5 min 16 s |

A structural diagram of a single crystal of the crystal form A was shown in FIG. 16. Data of the single crystal: monoclinic system, space group P2(1), unit cell parameters: a=10.2499(10) Å, b=17.9931(16) Å, c= 11.2407(8) Å, α=γ=90° β=93.349(8)°, deviation factor R1=0.0562, and Z=4.

### Crystal Form Example 2 Preparation of amorphous form

400 mg of the crystal form A prepared in Example 1 was weighed, dissolved in 20 mL of dichloromethane at room temperature and filtered, and then the filtrate was subjected to rotary evaporation to rapidly remove the solvent. The resulting solid sample was subjected to a corresponding characterization test. An XRD pattern of the sample can be seen in FIG. 8. After the amorphous sample was placed under ambient conditions (room temperature, >20%RH) overnight, a PLM thereof was observed again, and it was found that some sample had transformed into a crystal form. After this sample was further placed under the same conditions for two days, XRD results showed that the crystal form of the transformed sample was basically consistent with the crystal form A. The above results indicated that the amorphous sample was unstable under ambient conditions and recrystallized into the crystal form A after moisture absorption.

### Crystal Form Example 3 Preparation of crystal form E

A crystal form E sample was obtained by suspending and stirring the amorphous sample prepared in Example 2 in a 1,4-dioxane/n-heptane solvent system at room temperature. XRD results of the sample can be seen in FIG. 9. The results indicated that the crystal form E sample was transformed into the crystal form A after being air-dried during open storage at room temperature for approximately 1 day. On this basis, it was inferred that the crystal form E was a metastable crystal form.

### Crystal Form Example 4 Preparation of crystal form B

A crystal form B sample was obtained by purging the starting crystal form A sample under nitrogen (approximately 5%RH) for 2 hours. XRD results of the sample can be seen in FIG. 17. Since the sample was rapidly transformed into a crystal form C at room humidity (17.3%RH) on the same day, it was inferred that the crystal form B was a metastable crystal form.

### Crystal Form Example 5 Preparation of crystal form C

A crystal form C sample was obtained by purging the starting crystal form A sample under nitrogen (approximately 5%RH) for 2 hours and then placing at room humidity (17.3%RH) for 2 hours. XRD results of the sample can be seen in FIG. 18. TGA/DSC results can be seen in FIG. 19. The results indicated that the sample undergone a weight loss of 3.2% upon heating from room temperature to 150°C, with endothermic peaks at 57.5°C, 94.5°C and 138.4°C (peak temperatures). An ¹H NMR spectrum of the crystal form C was obtained by a test in DMSO-d6, and no obvious solvent residue was observed. Since the crystal form C was obtained by increasing the humidity of the crystal form B, combined with a distinct stepwise weight loss in a TGA curve and an endothermic signal in a DSC curve, it was preliminarily inferred that the crystal form C was a hydrate. The crystal form C was repeatedly prepared, but pure crystal form C could not be obtained.

### Crystal Form Example 6 Preparation of crystal form D

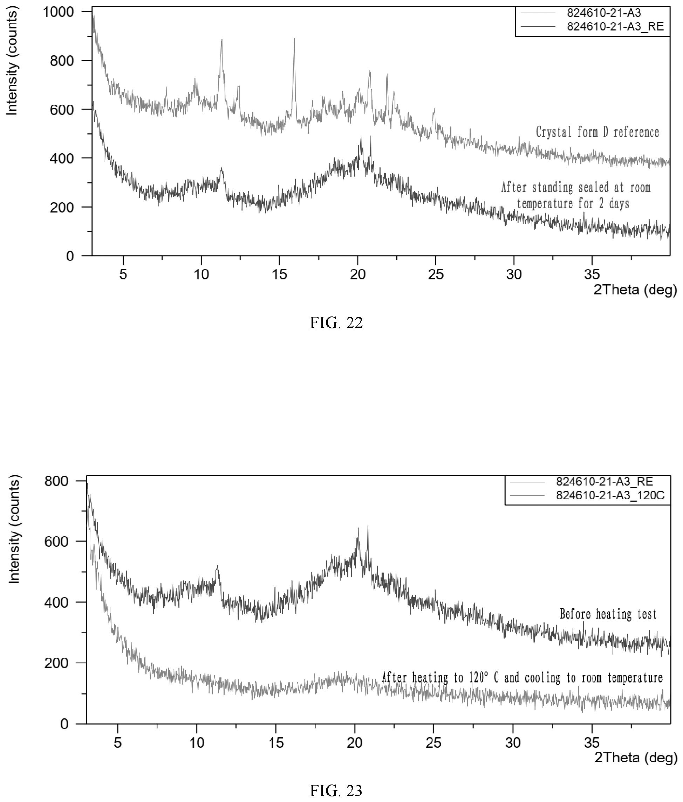

A crystal form D sample was obtained by slowly evaporating the starting crystal form A sample in a methanol solvent. XRD results of the sample can be seen in FIG. 20. TGA/DSC results can be seen in FIG. 21. The results indicated that the sample undergone a weight loss of 2.6% upon heating from room temperature to 150°C, with an endothermic peak at 83.4°C (peak temperature). An ¹H NMR spectrum of the crystal form D was obtained by a test in DMSO-d6, and no obvious solvent residue was observed. As shown in FIG. 22, a crystallinity of the crystal form D was significantly reduced after standing sealed at room temperature for 2 days. As shown in FIG. 23, the sample was transformed into an amorphous form after heating to 120°C and cooling to room temperature. Based on nuclear magnetic resonance data and heating test results of the sample, it was inferred that the crystal form D was a hydrate crystal form.

### Crystal Form Test Example 1 Evaluation of solubility and hygroscopicity of crystal form

Approximate solubilities of crystal form A in various organic solvents at room temperature were as follows:

| Solvent | Solubility (mg/mL) |
|---|---|
| Methanol | S>36.0 |
| Ethanol | S>38.0 |
| Isopropanol | S>42.0 |
| Dichloromethane | S>40.0 |
| Trichloromethane | S>44.0 |
| Acetonitrile | S>44.0 |
| Acetone | S>40.0 |
| Methyl isobutyl ketone | S>40.0 |
| Ethyl acetate | S>44.0 |
| Isopropyl acetate | S>38.0 |

Dynamic vapor sorption (DVS) was used to investigate hygroscopicities of an active pharmaceutical ingredient in different crystal forms.

Determination method:
instrument: dynamic vapor sorption analyzer; temperature: 25°C; protective gas and flow rate: N₂, 200 mL/min;
dm/dt: 0.002%/min; RH range: 0%RH-100%RH; cycle: 1 full cycle.

### Experimental results:

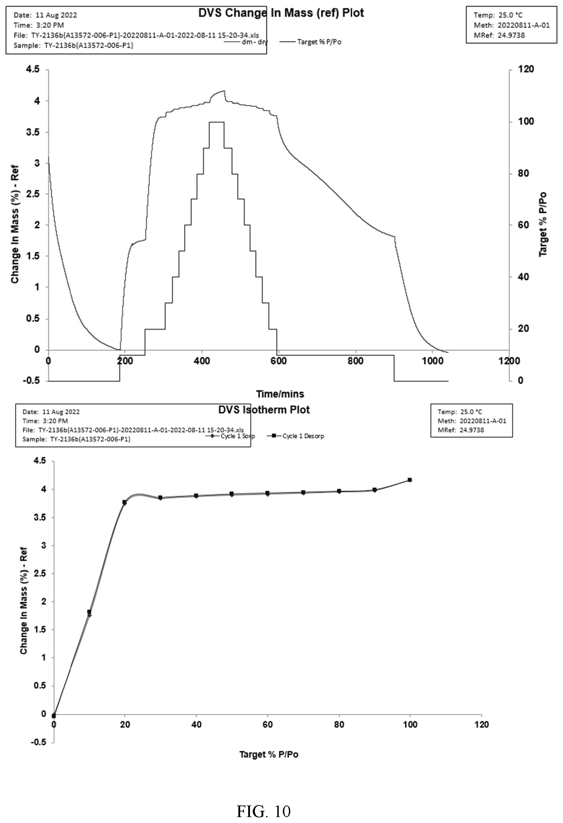

DVS experiment results of the crystal form A can be seen in FIG. 10. It can be seen from FIG. 10 that the weight loss of the crystal form A was approximately 3-4% at 0% humidity, which was consistent with a mass of water of crystallization. There was significant weight gain when the humidity was increased from 0% to 20%, there was almost no hygroscopic weight gain when the humidity was increased from 20% to 80%, and there was obvious hygroscopicity when the humidity was 90% or more. There was continuous weight loss when the humidity was reduced from 100% to 0%, and weight change trajectories during adsorption and desorption were completely overlapped.

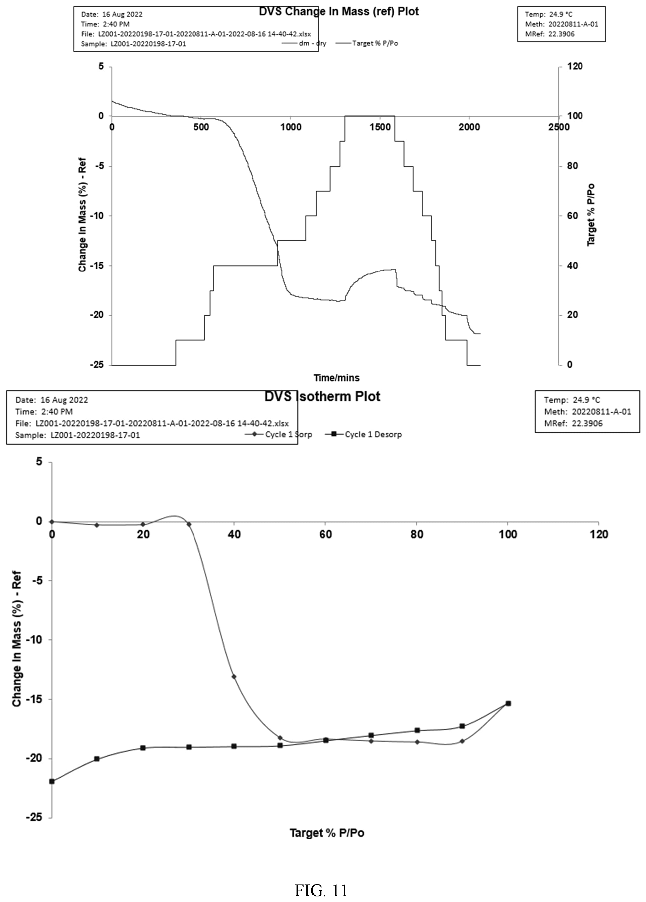

DVS experiment results of the crystal form E can be seen in FIG. 11. It can be seen from FIG. 11 that the crystal form E undergone continuous weight loss when the humidity was increased from 0% to 90%: the weight loss was approximately 2% when the humidity was increased from 0% to 30%, and there was abrupt weight loss when the humidity was increased from 40% to 50%. The hygroscopic weight gain was approximately 4% when the humidity was increased from 90% to 100%; and the weight loss was approximately 2% when the humidity was reduced from 100% to 90%, so that the hygroscopic weight gain was greater than the weight loss. There was significant weight loss of up to 3% when the humidity was 20% or less. The weight change trajectories during adsorption and desorption were essentially non-overlapped.

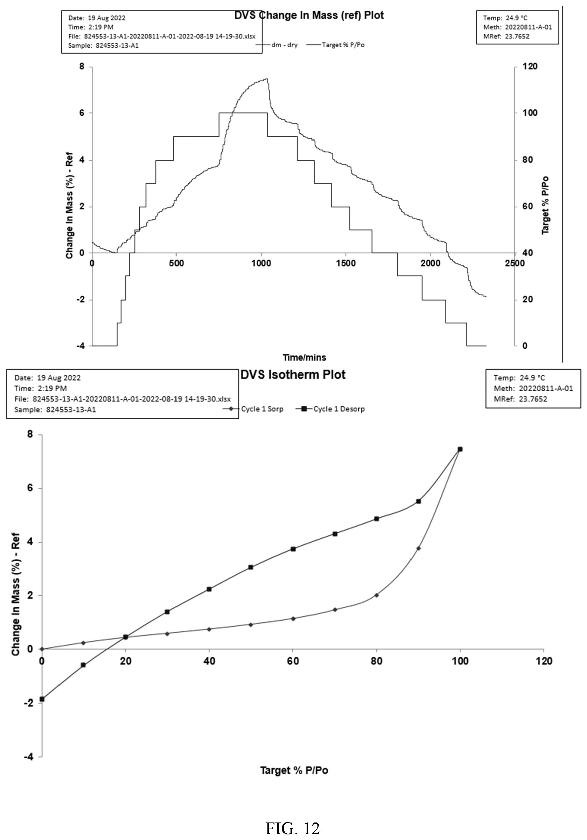

DVS experiment results of the amorphous form can be seen in FIG. 12. It can be seen from FIG. 12 that the amorphous sample undergone continuous weight gain when the humidity was increased from 0% to 100%: the weight gain was uniform in speed when the humidity was increased from 0% to 80%, and there was abrupt weight gain when the humidity was increased from 80% to 100%. The weight gain was approximately 3.5% when the humidity was increased from 90% to 100%; the amorphous sample undergone continuous weight loss when the humidity was reduced from 100% to 0%, the weight loss was rapid in speed when the humidity was reduced from 100% to 90%, the weight loss was approximately 1.5%, and the weight loss was smaller than the weight gain; and the weight loss was uniform in speed when the humidity was reduced from 90% to 0%. The weight change trajectories during adsorption and desorption were completely non-overlapped.

In conclusion, the crystal form A was a monohydrate, which did not lose water and did not have hygroscopicity under conventional conditions; the crystal form E is a solvate, which could lose an incorporated solvent thereof under normal ambient humidity variations and had hygroscopicity; and the amorphous form had hygroscopicity. Accordingly, the stability of the crystal form A under normal storage and production conditions was clearly better than those of the crystal form E and the amorphous form.

### Crystal Form Test Example 2 Evaluation of crystal form transformation relationship

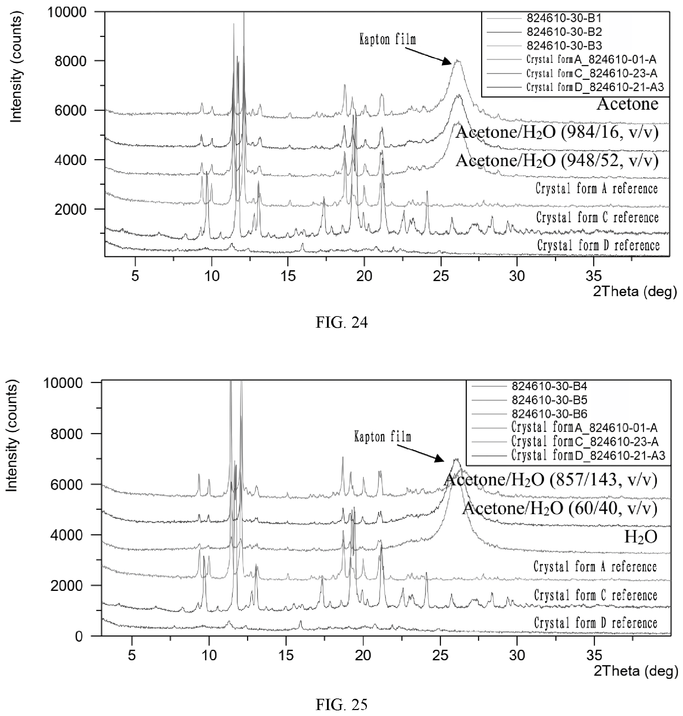

For crystal form A/C/D samples, which are relatively stable at room temperature, tthe transformation relationship among the crystal forms was investigated by suspension competition experiments under different water activities at room temperature. The starting crystal form A sample was vacuum-dried at room temperature and used for preparing saturated solutions with different water activities in an acetone/water system. After pre-equilibration for 8 to 24 hours, the solutions were filtered through a 0.45 µm PTFE filter membrane, and the filtrates were transferred to HPLC vials containing the crystal form A sample, the crystal form C sample and the crystal form D sample respectively. After suspension and stirring at room temperature, solid wet samples were separated and subjected to XRD testing (film-covered testing). The results are summarized in Table 10, and the XRD patterns can be seen in FIG. 24 and FIG. 25. The results demonstrated that the crystal form A was obtained in all systems.

**Table 10 Table of suspension competition experiments among crystal forms A/C/D**

| Starting crystal form | Serial No. | Solvent (v/v) | Temperature | Suspension time | Result |
|---|---|---|---|---|---|
| | A1 | Acetone | | 4 days | Crystal form A |
| | A2 | Acetone/water (984/16, aw-0.2) | | 3 days | Crystal form A |
| Crystal forms A/C/D | A3 | Acetone/water (948/52, aw-0.4) | Room temperature | 3 days | Crystal form A |
| | A4 | Acetone/water (857/143, aw-0.6) | | 4 days | Crystal form A |
| | A5 | Acetone/water (60/40, aw-0.8) | | 5 days | Crystal form A |
| | A6 | Water | | 6 days | Crystal form A |

### Crystal Form Test Example 3 Evaluation of physicochemical stability

To verify stabilities of crystal form A, crystal form E and the amorphous form, high-temperature, high-humidity and accelerated tests and a light exposure test were also carried out, and specific test methods and results can be seen below.

### 3.1 High-temperature test

An investigation method for the high-temperature test can be seen in Table 11 below.

**Table 11**

| Active pharmaceutical ingredient of the compound represented by formula I | Crystal form A | Crystal form E | Amorphous form |
|---|---|---|---|
| Amount stored (mg) | 502.00 | 129.98 | 501.07 |
| Storage condition | 50°C±2°C | | |
| Actual investigation time point | 0 day, 5 days, 11 days and 32 days | | |
| Packaging | Weighing bottle (open) | | |

### 3.2 High-humidity test

An investigation method for the high-humidity test can be seen in Table 12 below.

**Table 12**

| Active pharmaceutical ingredient of the compound represented by formula I | Crystal form A | Crystal form E | Amorphous form |
|---|---|---|---|
| Amount stored | 504.79 | 131.39 | 508.32 |
| Storage condition | 90%RH±5%RH | | |
| Actual investigation time point | 0 day, 5 days, 11 days and 32 days | | |
| Packaging | Weighing bottle (open) | | |

### 3.3 Light exposure test

An investigation method for the light exposure test can be seen in Table 13 below.

**Table 13**

| Active pharmaceutical ingredient of the compound represented by formula I | Crystal form A | Crystal form E | Amorphous form |
|---|---|---|---|
| Amount stored | 500.53 | 131.34 | 500.91 |
| Storage condition | 4500lx±500lx | | |
| Actual investigation time point | 0 day, 5 days, 11 days and 32 days | | |
| Packaging | Weighing bottle (open) | | |

### 3.4 Accelerated test

An investigation method for the accelerated test can be seen in Table 14 below.

**Table 14**

| Active pharmaceutical ingredient of compound represented by formula I | Crystal form A | Crystal form E | Amorphous form |
|---|---|---|---|
| Amount stored | 503.60 | 132.12 | 508.32 |
| Storage condition | 40°C±2°C, 75%RH±5%RH | | |
| Actual investigation time point | 0 day, 5 days, 11 days and 32 days | | |
| Packaging | Weighing bottle (open) | | |

Results of related substances in each crystal form under the high-temperature condition (50°C±2°C) can be seen in Table 15.

HPLC detection results of related substances under the high-temperature condition:

**Table 15**

| Component | Impurity content in crystal form A (%) | | | | Impurity content in crystal form E (%) | | | |
|---|---|---|---|---|---|---|---|---|
| | 0 day | 5 days | 11 days | 32 days | 0 day | 5 days | 11 days | 32 days |
| RRT0.55 | 0.03 | 0.03 | 0.03 | 0.05 | 0.04 | 0.03 | 0.04 | 0.04 |
| RRT0.66 | 0.18 | 0.19 | 0.19 | 0.20 | 0.21 | 0.22 | 0.22 | 0.26 |
| RRT0.71 | 0.15 | 0.16 | 0.16 | 0.17 | 0.14 | 0.15 | 0.13 | 0.14 |
| RRT0.94 | 0.04 | 0.04 | 0.03 | 0.05 | 0.03 | 0.03 | 0.03 | 0.05 |
| RRT1.02 | 0.28 | 0.31 | 0.30 | 0.30 | 0.21 | 0.27 | 0.30 | 0.40 |
| RRT1.06 | 0.07 | 0.08 | 0.08 | 0.08 | 0.07 | 0.05 | 0.06 | 0.05 |
| RRT1.08 | 0.16 | 0.19 | 0.22 | 0.21 | 0.17 | 0.24 | 0.29 | 0.40 |
| RRT1.15 | 0.03 | 0.03 | 0.03 | 0.05 | 0.03 | 0.03 | 0.03 | 0.05 |
| Total impurities (%) | 0.95 | 1.03 | 1.04 | 1.11 | 0.89 | 1.03 | 1.10 | 1.40 |

After being stored under the high-temperature condition (50°C±2°C) for 32 days, the content of the specific impurity RRT0.66 in the crystal form A showed no obvious change with a prolonged high-temperature duration (increase ≤ 0.02%), while the content of the impurity in the crystal form E was increased by 0.05%. The contents of the specific impurity RRT0.71 in the two crystal forms showed no obvious changes with the prolonged high-temperature duration. The content of the specific impurity RRT1.02 in the crystal form A showed no obvious change, while the content of the impurity in the crystal form E was increased by 0.19%. The contents of the specific impurity RRT1.08 in the crystal form A and the crystal form E were increased by 0.05% and 0.23% respectively. The contents of other non-specified impurities were all not more than 0.10%. The contents of total impurities in the crystal form A and the crystal form E were increased by 0.16% and 0.51% respectively, both of which were not more than 2.0%. In summary, the stability of the crystal form A was better than that of the crystal form E under the high-temperature condition.

Results of related substances in each crystal form under the high-humidity condition (90%RH±5%RH) can be seen in Table 16.

HPLC detection results of related substances under the high-humidity condition can be seen as follows:

**Table 16**

| Component | Impurity content in crystal form A (%) | | | | Impurity content in crystal form E (%) | | | | Impurity content in amorphous form (%) | | | |
|---|---|---|---|---|---|---|---|---|---|---|---|---|
| | 0 day | 5 days | 11 days | 32 days | 0 day | 5 days | 11 days | 32 days | 0 day | 5 days | 11 days | 32 days |
| RRT0.55 | 0.03 | 0.03 | 0.03 | 0.03 | 0.04 | 0.03 | 0.04 | 0.04 | 0.03 | 0.03 | 0.03 | 0.03 |
| RRT0.66 | 0.18 | 0.20 | 0.20 | 0.18 | 0.21 | 0.21 | 0.21 | 0.22 | 0.20 | 0.21 | 0.20 | 0.20 |
| RRT0.71 | 0.15 | 0.16 | 0.16 | 0.16 | 0.14 | 0.15 | 0.14 | 0.15 | 0.16 | 0.17 | 0.16 | 0.18 |
| RRT0.94 | 0.04 | 0.03 | 0.03 | 0.05 | 0.03 | 0.03 | 0.02 | 0.05 | 0.04 | 0.03 | 0.03 | 0.04 |
| RRT1.02 | 0.28 | 0.31 | 0.30 | 0.26 | 0.21 | 0.21 | 0.20 | 0.19 | 0.29 | 0.31 | 0.31 | 0.31 |
| RRT1.06 | 0.07 | 0.09 | 0.08 | 0.06 | 0.07 | 0.05 | 0.05 | 0.04 | 0.07 | 0.09 | 0.09 | 0.06 |
| RRT1.08 | 0.16 | 0.21 | 0.21 | 0.18 | 0.17 | 0.18 | 0.21 | 0.21 | 0.18 | 0.19 | 0.25 | 0.23 |
| RRT1.15 | 0.03 | 0.04 | 0.03 | 0.05 | 0.03 | 0.03 | 0.03 | 0.04 | 0.04 | 0.04 | 0.03 | 0.05 |
| Total impurities (%) | 0.95 | 1.07 | 1.05 | 0.98 | 0.89 | 0.89 | 0.89 | 0.94 | 1.01 | 1.07 | 1.11 | 1.11 |

After being stored under the high-humidity condition (90%RH±5%RH) for 32 days, the contents of the specific impurities RRT0.66 and RRT0.71 in all the three crystal forms showed no essential changes (increase ≤ 0.02%). The contents of the specific impurity RRT1.02 in the crystal form A and the crystal form E showed no essential changes, while the content of the impurity in the amorphous form was increased by 0.03%. The content of the specific impurity RRT1.08 in the crystal form A showed no essential change, while the contents of the impurity in the crystal form E and the amorphous form were increased by 0.04% and 0.05% respectively. The contents of other non-specified impurities were all not more than 0.10%. The contents of total impurities in the crystal form A, the crystal form E and the amorphous form were increased by 0.03%, 0.05% and 0.10% respectively, all of which were not more than 2.0%. In summary, the stability of the crystal form A was better than those of the crystal form E and the amorphous form under high-humidity condition.

Results of related substances in each crystal form under the light exposure condition (4500lx±500lx) can be seen in Table 17.

HPLC detection results of related substances under the light exposure condition can be seen as follows:

**Table 17**

| Component | Impurity content in crystal form A (%) | | | | Impurity content in crystal form E (%) | | | | Impurity content in amorphous form (%) | | | |
|---|---|---|---|---|---|---|---|---|---|---|---|---|
| | 0 day | 5 days | 11 days | 32 days | 0 day | 5 days | 11 days | 32 days | 0 day | 5 days | 11 days | 32 days |
| RRT0.55 | 0.03 | 0.03 | 0.03 | 0.03 | 0.04 | 0.03 | 0.04 | 0.03 | 0.03 | 0.04 | 0.03 | 0.05 |
| RRT0.66 | 0.18 | 0.21 | 0.22 | 0.27 | 0.21 | 0.22 | 0.22 | 0.25 | 0.20 | 0.20 | 0.21 | 0.23 |
| RRT0.71 | 0.15 | 0.14 | 0.15 | 0.10 | 0.14 | 0.14 | 0.14 | 0.13 | 0.16 | 0.17 | 0.17 | 0.21 |
| RRT0.92 | N.D | | | | N.D | | 0.03 | N.D | N.D | | | |
| RRT0.94 | 0.04 | 0.04 | 0.03 | 0.07 | 0.03 | 0.02 | 0.03 | 0.08 | 0.04 | 0.03 | 0.03 | 0.06 |
| RRT1.02 | 0.28 | 0.30 | 0.31 | 0.28 | 0.21 | 0.22 | 0.20 | 0.20 | 0.29 | 0.32 | 0.33 | 0.35 |
| RRT1.06 | 0.07 | 0.08 | 0.07 | 0.06 | 0.07 | 0.05 | 0.04 | 0.04 | 0.07 | 0.08 | 0.09 | 0.07 |
| RRT1.08 | 0.16 | 0.20 | 0.22 | 0.25 | 0.17 | 0.20 | 0.24 | 0.25 | 0.18 | 0.21 | 0.28 | 0.37 |
| RRT1.15 | 0.03 | 0.04 | 0.04 | 0.02 | 0.03 | 0.04 | 0.03 | 0.04 | 0.04 | 0.04 | 0.03 | 0.05 |
| Total impurities (%) | 0.95 | 1.03 | 1.08 | 1.08 | 0.89 | 0.92 | 0.98 | 1.02 | 1.01 | 1.09 | 1.17 | 1.38 |

| | | | | | | | | | | | | |
|---|---|---|---|---|---|---|---|---|---|---|---|---|
| Note: N.D. indicated "not detected". | | | | | | | | | | | | |

After being stored under the light exposure condition (4500 lx±500 lx) for 32 days, the contents of the specific impurity RRT0.66 in the crystal form A, the crystal form E and the amorphous form were increased by 0.09%, 0.04% and 0.03% respectively. The contents of the specific impurities RRT0.71 and RRT1.02 in the crystal form A and the crystal form E were not increased, while the contents of the impurities in the amorphous form were increased by 0.05% and 0.06% respectively. The contents of the specific impurity RRT1.08 in the crystal form A, the crystal form E and the amorphous form were increased by 0.09%, 0.08% and 0.19% respectively. The contents of other non-specified impurities were all not more than 0.10%. The contents of total impurities in the crystal form A, the crystal form E and the amorphous form were increased by 0.13%, 0.13% and 0.37% respectively, all of which were not more than 2.0%. In summary, the stabilities of the crystal form A and the crystal form E were better than that of the amorphous form under the light exposure condition.

Change results of related substances in each crystal form under the accelerated condition (40°C±2°C, 75%RH±5%RH) can be seen in Table 18.

HPLC detection results of related substances under the accelerated condition can be seen as follows:

**Table 18**

| Component | Impurity content in crystal form A (%) | | | | Impurity content in crystal form E (%) | | | | Impurity content in amorphous form (%) | | | |
|---|---|---|---|---|---|---|---|---|---|---|---|---|
| | 0 day | 5 days | 11 days | 32 days | 0 day | 5 days | 11 days | 32 days | 0 day | 5 days | 11 days | 32 days |
| RRT0.55 | 0.03 | 0.03 | 0.04 | 0.04 | 0.04 | 0.04 | 0.04 | 0.04 | 0.03 | 0.04 | 0.03 | 0.04 |
| RRT0.66 | 0.18 | 0.20 | 0.20 | 0.20 | 0.21 | 0.21 | 0.21 | 0.21 | 0.20 | 0.20 | 0.20 | 0.20 |
| RRT0.71 | 0.15 | 0.16 | 0.17 | 0.16 | 0.14 | 0.15 | 0.15 | 0.16 | 0.16 | 0.17 | 0.17 | 0.18 |
| RRT0.94 | 0.04 | 0.04 | 0.03 | 0.04 | 0.03 | 0.03 | 0.02 | 0.04 | 0.04 | 0.03 | 0.03 | 0.04 |
| RRT1.02 | 0.28 | 0.30 | 0.33 | 0.27 | 0.21 | 0.21 | 0.21 | 0.20 | 0.29 | 0.32 | 0.31 | 0.29 |
| RRT1.06 | 0.07 | 0.08 | 0.09 | 0.07 | 0.07 | 0.05 | 0.05 | 0.04 | 0.07 | 0.08 | 0.08 | 0.06 |
| RRT1.08 | 0.16 | 0.18 | 0.25 | 0.20 | 0.17 | 0.18 | 0.21 | 0.20 | 0.18 | 0.22 | 0.24 | 0.21 |
| RRT1.15 | 0.03 | 0.03 | 0.04 | 0.06 | 0.03 | 0.03 | 0.03 | 0.05 | 0.04 | 0.04 | 0.03 | 0.03 |
| Total impurities (%) | 0.95 | 1.02 | 1.14 | 1.03 | 0.89 | 0.89 | 0.94 | 0.95 | 1.01 | 1.09 | 1.10 | 1.05 |

After being stored under the accelerated condition (40°C±2°C, 75%RH±5%RH) for 32 days, the contents of the specific impurities RRT0.66, RRT0.71 and RRT1.02 in all the three crystal forms showed no obvious changes (increase ≤ 0.02%). The contents of the specific impurity RRT1.08 in the crystal form A, the crystal form E and the amorphous form were increased by 0.04%, 0.03% and 0.03% respectively, with comparable increases. The contents of other non-specified impurities were all not more than 0.10%. The contents of total impurities were all not more than 2.0%. It was indicated that the three crystal forms exhibit comparable stabilities under the accelerated condition.

Under the high-temperature (50°C±2°C) and high-humidity (90%RH±5%RH) conditions, the stability of the crystal form A was better than those of the crystal form E and the amorphous form respectively. Under the light exposure condition (4500lx±500lx), the stabilities of the crystal form A and the crystal form E were both better than that of the amorphous form. Under the accelerated condition (40°C±2°C, 75%RH±5%RH), the three crystal forms exhibit comparable stabilities. Compared with other conditions, under the light exposure condition (4500lx±500lx), the three crystal forms showed poorer stabilities, which suggested that storage in the dark was required.

In conclusion, compared with the crystal form E and the amorphous form, the crystal form A was the most stable crystal form and was the most suitable crystal form for subsequent processing and development.

### Crystal Form Test Example 4 Flowability test

A flowability determination method was as follows: an angle of repose was determined by using a device composed of 2 to 3 funnels connected in series in a staggered manner, and drug powder was slowly and uniformly passed through the funnels onto a stationary base with a diameter d, so as to form a symmetric powder pile with a single layer of powder at the bottom. During the formation of the powder pile, a height of the funnels was maintained within 2-4 cm from a top of the powder pile, a height h of the cone was determined, and the angle of repose was calculated as tanθ=h/(d/2).

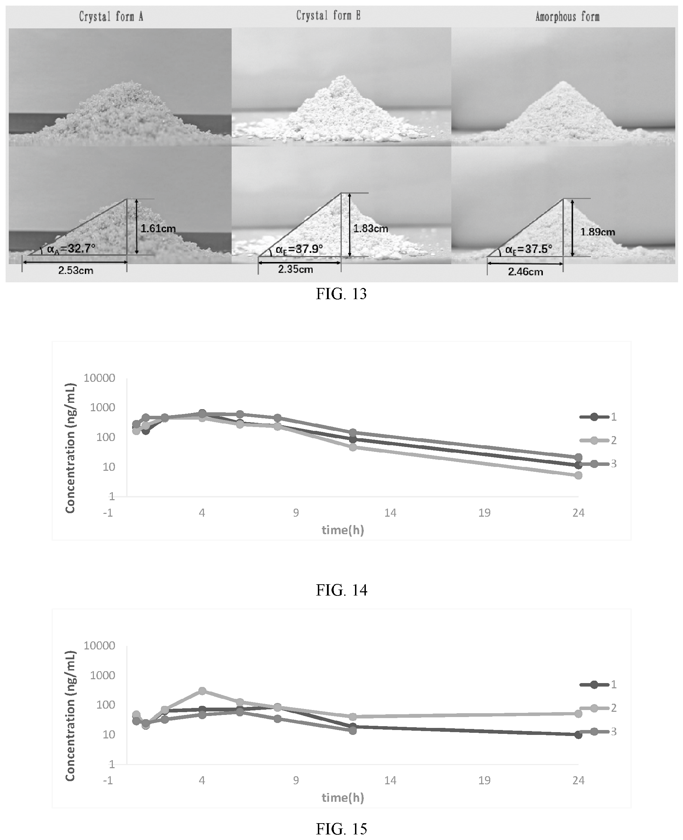

Experimental results: powder flowability test results of three crystal forms of an active pharmaceutical ingredient can be seen in FIG. 13.

According to the test, an angle of repose of the crystal form A sample was approximately 32.7°, an angle of repose of the crystal form E sample was 37.9°, and an angle of repose of the amorphous sample was approximately 37.5°.

Since a smaller angle of repose indicated a better powder flowability, a flowability of the crystal form A was better than those of the crystal form E and the amorphous form.

### Crystal Form Test Example 5 Pharmacokinetic test

This test example comparatively investigated pharmacokinetic processes of a crystal form A and an amorphous form in SD rats, and compared pharmacokinetic characteristics of the forms in the SD rats.
Experimental methods and materials
Compound information
Test article
Name: compound represented by formula I (crystal form A)
Name: compound represented by formula I (amorphous form)
Internal standard
Name: verapamil

Experimental animals: healthy adult SD rats, male, 6 in total, divided into two groups, with 3 rats per group, aged 6-8 weeks; and body weight approximately 200-300 g. The rats were fed in rat cages, and fasted (for at least 10 hours) with free access to water one day before the experiment. On the day of experiment, the rats were weighed and marked at the tail respectively. Blank blood samples were collected respectively before drug administration. The blood samples were collected via the tail vein. Drug administration method: intragastric gavage (p.o.): intragastric gavage with drug suspension; preparation of drug suspension: approximately 10 mg of test sample was accurately weighed, dissolved in 5% DMSO (calculated proportion), and vortexed with 10% solutol HS-15 and 85% saline to obtain a suspension with a concentration of 1.0 mg/mL; and the suspension was freshly prepared before use.

Sample collection: the rats were administered a single oral dose of 10mg/kg via intragastric gavage (p.o.). Timing was started immediately after the administration was completed, and blood samples were collected at 0.5, 1, 2, 4, 6, 8, 12 and 24 hours after administration. A volume of 0.1mL of whole blood was collected into EDTA-Na₂ anticoagulant tubes, mixed by inverting 3-4 times, centrifuged at 10000 g for 5 minutes at 4°C to separate the plasma, and then the plasma was stored at -80 °C until testing. The blood samples were collected via the tail vein.

Sample preparation: 1) the samples were thawed, and 15 µL of unknown plasma samples before or after administration, standard series solutions, and single blank and double blank samples were respectively transferred into 1.5 mL centrifuge tubes. 2) 15 µL of protein precipitant (methanol) and 400 µL of internal standard solution (verapamil in methanol, approximately 10 ng/mL) were added to each sample sequentially, vortexed for 2 minutes, and then centrifuged at 4°C and 12000 rpm for 10 minutes. 3) The supernatant was collected for LC-MS/MS detection.

Analytical conditions: liquid chromatography conditions: liquid chromatography system: Shimadzu Nexera X2; chromatographic column: Agilent ZORBAX XDB-C18 3.5 (2.1 × 50 mm); column temperature: 35°C; mobile phase: A-5% acetonitrile (0.1% formic acid in water), B-95% acetonitrile (0.1% formic acid in water); injection volume: 3 µL; and

flow rate: 0.5 mL/min. Gradient elution was employed, and an elution program can be seen in the table below.

**Table 19**

| Time (min) | 0.01 | 0.2 | 1.7 | 2.0 | 2.0 | 2.5 |
|---|---|---|---|---|---|---|
| % B | 10 | 10 | 100 | 100 | 10 | 10 |

Mass spectrometry conditions: mass spectrometric analysis was carried out using an electrospray ionization (ESI) source in a positive ion mode with multiple reaction monitoring (MRM). The mass spectrometer ion source parameters and compound detection parameters can be seen in the table below.

Mass spectrometer ion source parameters

**Table 20**

| Instrument | Triple QuadTM 6500+AB mass spectrometry system, manufactured in Canada |
|---|---|
| Ion source mode | ESI |
| Scanning mode | MRM |
| Curtain gas | 20 L/min |
| Nebulizer gas | 50 L/min |
| Auxiliary gas | 50 L/min |
| Ion source temperature | 300°C |
| Ion spray voltage | + 5500 v (positive MRM) |

The Main scan parameters for the analyte and internal standard can be seen in the table below.

**Table 21**

| Name of compound | Q1 (m/z) | Q3 (m/z) | DP (v) | EP (v) | CE (v) | CXP (v) |
|---|---|---|---|---|---|---|
| Compound represented by formula I | 391.1 | 159.1 | 135 | 10 | 50 | 15 |
| IS (verapamil) | 455.3 | 165.2 | 135 | 10 | 35 | 15 |

PK parameter processing: pharmacokinetic (PK) parameters were calculated according to a plasma concentration from individual plasma concentration and corresponding sampling time data using Phoenix WinNonlin software with a non-compartmental model. (1) The PK parameters used for pharmacokinetic evaluation included: Cmax, AUC0-t, AUC0-∞, Tmax and t1/2.

(2) The parameters used for bioequivalence evaluation: Cmax, AUC0-t and AUC0-∞ were used for the bioequivalence evaluation.

Cmax: maximum plasma concentration measured within specified period, presented as measured value.

Tmax: time measured to reach maximum plasma concentration, presented as measured value. If the maximum value occurred at more than one time point, Tmax was defined as the first time point at which this value was observed.

AUC0-t: area under plasma concentration-time curve from 0 to last time point t.

AUC0-∞: area under plasma concentration-time curve from 0 to infinity. Ct was the last measured concentration, and λz was the terminal elimination rate constant. ${AUC}_{0 - ∞} = {AUC}_{0 - t} + \frac{Ct}{λz} {AUC}_{0 - ∞} = {AUC}_{0 - t} + \frac{Ct}{λz}$

t1/2: elimination or terminal half-life, estimated by ln2/λz.

Results: the linear range of the compound represented by formula I was 2-2000 ng/ml. Following administration of 10 mg/kg of the compound represented by formula I (crystal form A) and the compound represented by formula I (amorphous form) to rats, the plasma concentration-time profiles can be seen in Table 22 and Table 23, and FIG. 14 and FIG.15, respectively. The main pharmacokinetic parameters in rats can be seen in Tables 24 and 25.

**Table 22 Plasma concentration-time data of compound represented by formula I (crystal form A) in rats after administration at 10 mg/kg**

| Plasma concentration (ng/mL) | | | | | | |
|---|---|---|---|---|---|---|
| Time (h) | No. | | | Mean | SD | CV (%) |
| | 1 | 2 | 3 | | | |
| 0.5 | 220 | 168 | 285 | 224 | 58.6 | 26.1 |
| 1 | 170 | 257 | 474 | 300 | 157 | 52.1 |
| 2 | 448 | 453 | 470 | 457 | 11.5 | 2.52 |
| 4 | 652 | 459 | 623 | 578 | 104 | 18.0 |
| 6 | 307 | 277 | 602 | 395 | 180 | 45.4 |
| 8 | 240 | 238 | 459 | 312 | 127 | 40.7 |
| 12 | 88.3 | 47.2 | 146 | 93.8 | 49.6 | 52.9 |
| 24 | 11.6 | 5.25 | 21.3 | 12.7 | 8.08 | 63.6 |

**Table 23 Plasma concentration-time data of compound represented by formula I (amorphous form) in rats after administration at 10 mg/kg**

| Plasma concentration (ng/mL) | | | | | | |
|---|---|---|---|---|---|---|
| Time (h) | No. | | | Mean | SD | CV (%) |
| | 1 | 2 | 3 | | | |
| 0.5 | 44.5 | 49.0 | 29.4 | 41.0 | 10.3 | 25.1 |
| 1 | 20.8 | 21.5 | 25.3 | 22.5 | 2.42 | 10.7 |
| 2 | 64.1 | 71.2 | 33.2 | 56.2 | 20.2 | 36.0 |
| 4 | 71.7 | 307 | 47.9 | 142 | 143 | 101 |
| 6 | 72.4 | 128 | 58.3 | 86.2 | 36.9 | 42.7 |
| 8 | 87.1 | 84.3 | 34.7 | 68.7 | 29.5 | 42.9 |
| 12 | 18.6 | 40.6 | 14.0 | 24.4 | 14.2 | 58.3 |
| 24 | 10.2 | 52.2 | NA | 31.2 | 29.7 | 95 |

**Table 24 Some pharmacokinetic parameters of compound represented by formula I (crystal form A) in rats after administration by intragastric gavage at 10 mg/kg**

| No. | Tmax (h) | Cmax (ng/ml) | AUClast (h*ng/ml) | AUCinf (h*ng/ml) | HL_ (h) | MRTlast(h) |
|---|---|---|---|---|---|---|
| 1 | 4.00 | 652 | 4324 | 4386 | 3.76 | 6.09 |
| 2 | 4.00 | 459 | 3551 | 3575 | 3.07 | 5.52 |
| 3 | 4.00 | 623 | 6326 | 6440 | 3.70 | 6.66 |
| Mean | 4.00 | 578 | 4734 | 4800 | 3.51 | 6.09 |
| SD | 0.00 | 104 | 1432 | 1477 | 0.38 | 0.57 |
| CV (%) | 0.00 | 18.0 | 30.3 | 30.8 | 10.9 | 9.41 |

**Table 25 Some pharmacokinetic parameters of compound represented by formula I (amorphous form) in rats after administration by intragastric gavage at 10 mg/kg**

| No. | Tmax (h) | Cmax (ng/ml) | AUClast (h*ng/ml) | AUCinf (h*ng/ml) | HL_ (h) | MRTlast(h) |
|---|---|---|---|---|---|---|
| 1 | 8.00 | 87.1 | 894 | 982 | 6.05 | 7.84 |
| 2 | 4.00 | 307 | 1908 | 3203 | 17.2 | 9.26 |
| 3 | 6.00 | 58.3 | 428 | 487 | 2.93 | 5.56 |
| Mean | 6.00 | 151 | 1077 | 1558 | 8.72 | 7.55 |
| SD | 2.00 | 136 | 757 | 1446 | 7.50 | 1.87 |
| CV (%) | 33.3 | 90.2 | 70.3 | 92.9 | 85.9 | 24.8 |

The values of Cmax in the rats after oral administration of the same dose of the compound represented by formula I (crystal form A) and the compound represented by formula I (amorphous form) were 578 ng/mL and 151 ng/mL respectively; and the values of AUClast in the rats were 4734 h*ng/ml and 1077h*ng/ml respectively, with a significant difference therebetween (p<0.05), which indicated that the bioavailability of the crystal form A was significantly higher than that of the amorphous form.

The embodiments of the present disclosure have been described above. However, the present disclosure is not limited to the above embodiments. Any modification, equivalent substitution, improvement, etc. made within the spirit and principle of the present disclosure shall be included in the scope of protection of the present disclosure. All documents mentioned in the present disclosure are incorporated by reference in the present disclosure, just as each document is individually incorporated by reference. In addition, it should be understood that, after reading the above contents explained in the present disclosure, those skilled in the art can make various changes or modifications to the present disclosure, and these equivalent forms also fall within the scope defined by the appended claims of the present disclosure.

## Claims

1. A pharmaceutical composition, comprising:
crystal form A of a monohydrate of a compound represented by formula I 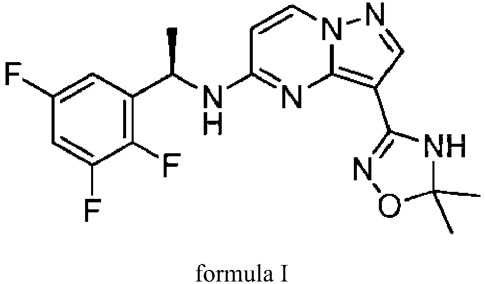
and a pharmaceutically acceptable carrier, adjuvant or excipient;
wherein, the crystal form A has one or more characteristic peaks at 9.35±0.2°, 11.42±0.2°, 12.06±0.2°, 18.71±0.2° and 21.16±0.2° in an X-ray powder diffraction pattern expressed in terms of 2θ angles;
preferably, the crystal form A has one or more characteristic peaks at 9.97±0.2°, 13.16±0.2°, 19.15±0.2°, 19.97±0.2° and 21.00±0.2° in the X-ray powder diffraction pattern expressed in terms of 2θ angles;
preferably, the crystal form A has the X-ray powder diffraction pattern substantially as shown in FIG. 5; and
more preferably, the pharmaceutical composition is prepared as an oral formulation.

2. The pharmaceutical composition according to claim 1, wherein the crystal form A has a thermogravimetric analysis thermogram and a differential scanning calorimetry thermogram substantially as shown in FIG. 6; and
preferably, the crystal form A belongs to a monoclinic system and belongs to a space group P2(1), with unit cell parameters: a=10.2499(10) Å, b=17.9931(16) Å, c= 11.2407(8) Å, α=γ=90° β=93.349(8)°, deviation factor R₁=0.0562, and Z=4.

3. The pharmaceutical composition according to claim 1,
wherein, the pharmaceutical composition comprises the following components in parts by weight:
| Component | Parts by weight |
|---|---|
| Crystal form A | 2.5-40 |
| First diluent | 10-70 |
| Second diluent | 10-70 |
| Glidant | 0.5-5 |
| Disintegrant | 1-15 |
| Lubricant | 0.2-10 |
| Coating material | 1-10 |
;
preferably, D90 of the crystal form A is 17-523 µm, more preferably 17-191 µm;
preferably, the first diluent is selected from mannitol, lactose, anhydrous calcium hydrogen phosphate, or a combination thereof;
preferably, the second diluent is microcrystalline cellulose;
preferably, the glidant is colloidal silicon dioxide;
preferably, the disintegrant is selected from sodium carboxymethyl starch, croscarmellose sodium, crospovidone, or a combination thereof;
preferably, the lubricant is selected from sodium stearyl fumarate, magnesium stearate, or a combination thereof; and
preferably, the coating material comprises a stabilizing substance and a polymer, wherein the stabilizing substance is at least one selected from titanium dioxide, talc and yellow iron oxide, and the polymer is at least one selected from polyvinyl alcohol and polyethylene glycol.

4. The pharmaceutical composition according to claim 1, wherein the pharmaceutical composition has one or more of the following characteristics:
1) a dissolution of the crystal form A in the pharmaceutical composition is ≥70% within 15 minutes under conditions of pH 6.0-7.6 and 0.2% SDS;
2) a dissolution of the crystal form A in the pharmaceutical composition is ≥85% within 45 minutes under conditions of pH 6.0-7.6 and 0.2% SDS;
3) a dissolution of the crystal form A in the pharmaceutical composition is ≥95% within 60 minutes under conditions of pH 6.0-7.6 and 0.2% SDS;
4) a dissolution of the crystal form A in the pharmaceutical composition is ≥85% within 45 minutes after the pharmaceutical composition is stored for 18 months under conditions of 25±2°C and 60%±5%RH; and
5) a dissolution of the crystal form A in the pharmaceutical composition is ≥85% within 45 minutes after the pharmaceutical composition is stored for 6 months under conditions of 40±2°C and 75%±5%RH.

5. A preparation method for the pharmaceutical composition according to any one of claims 1 to 4, comprising:
providing the following materials as raw materials in parts by weight, and preparing the raw materials into the composition:
| Component | Parts by weight |
|---|---|
| Crystal form A | 2.5-40 |
| First diluent | 10-70 |
| Second diluent | 10-70 |
| Glidant | 0.5-5 |
| Disintegrant | 1-15 |
| Lubricant | 0.2-10 |
| Coating material | 1-10 |
;
preferably,
| Component | Parts by weight |
|---|---|
| Crystal form A | 4-30 |
| First diluent | 20-60 |
| Second diluent | 20-60 |
| Glidant | 1-4 |
| Disintegrant | 2-12 |
| Lubricant | 0.5-10 |
| Coating material | 1.5-8 |
;
preferably, D90 of the crystal form A is 17-523 µm, more preferably 17-191 µm;
preferably, the first diluent is selected from mannitol, lactose, anhydrous calcium hydrogen phosphate, or a combination thereof;
preferably, the second diluent is microcrystalline cellulose;
preferably, the glidant is colloidal silicon dioxide;
preferably, the disintegrant is selected from sodium carboxymethyl starch, croscarmellose sodium, crospovidone, or a combination thereof;
preferably, the lubricant is selected from sodium stearyl fumarate, magnesium stearate, or a combination thereof;
preferably, the coating material comprises a stabilizing substance and a polymer, wherein the stabilizing substance is at least one selected from titanium dioxide, talc and yellow iron oxide, and the polymer is at least one selected from polyvinyl alcohol and polyethylene glycol;
preferably, the preparation method for the crystal form A comprises slurrying the compound represented by formula I in a mixed solvent of acetonitrile and water; and more preferably, the preparation method for the crystal form A comprises slurrying the compound represented by formula I in the mixed solvent of acetonitrile and water for 1-4 days, and centrifuging to collect a crystalline powder solid.

6. Crystal form A of a monohydrate of a compound represented by formula I 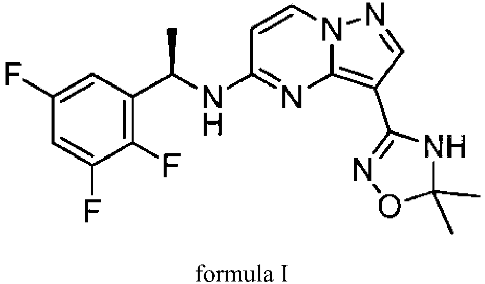
and a pharmaceutically acceptable carrier, adjuvant or excipient;
wherein, the crystal form A has one or more characteristic peaks at 9.35±0.2°, 11.42±0.2°, 12.06±0.2°, 18.71±0.2° and 21.16±0.2° in an X-ray powder diffraction pattern expressed in terms of 2θ angles;
preferably, the crystal form A has one or more characteristic peaks at 9.97±0.2°, 13.16±0.2°, 19.15±0.2°, 19.97±0.2° and 21.00±0.2° in the X-ray powder diffraction pattern expressed in terms of 20 angles; and
more preferably, the crystal form A has the X-ray powder diffraction pattern substantially as shown in FIG. 5.

7. A preparation method for the crystal form A according to claim 6, comprising slurrying the compound represented by formula I in a mixed solvent of acetonitrile and water, wherein, more preferably, the preparation method for the crystal form A comprises slurrying the compound represented by formula I in the mixed solvent of acetonitrile and water for 1-4 days, and centrifuging to collect a crystalline powder solid.

8. Use of the pharmaceutical composition according to any one of claims 1 to 5, or the crystal form A according to claim 6, in the manufacture of a medicament for the treatment and/or prevention of cancer or tumor, wherein, preferably, the cancer or tumor targeted by the medicament is selected from breast cancer, cervical cancer, colon cancer, lung cancer, gastric cancer, rectal cancer, pancreatic cancer, brain cancer, skin cancer, oral cancer, prostate cancer, bone cancer, renal cancer, ovarian cancer, bladder cancer, liver cancer, fallopian tube tumor, peritoneal tumor, melanoma, glioma, glioblastoma, head and neck cancer, papillary renal tumor, leukemia, lymphoma, myeloma and thyroid tumor.

9. Use of the pharmaceutical composition according to any one of claims 1 to 5, or the crystal form A according to claim 6, in the manufacture of a medicament for the treatment and/or prevention of a disease mediated by ROS1, NTRK or ALK, wherein, preferably, the disease mediated by ROS1, NTRK or ALK is selected from cancer, sarcoma and pain.
